(19) Europäisches Patentamt
European Patent Office
Office européen des brevets

(11) EP 2 294 068 B1

(12) # FASCICULE DE BREVET EUROPEEN

(45) Date de publication et mention de la délivrance du brevet:
**02.11.2011 Bulletin 2011/44**

(21) Numéro de dépôt: **09738348.3**

(22) Date de dépôt: **17.04.2009**

(51) Int Cl.:
***C07D 471/04*** *(2006.01)* ***A61K 31/437*** *(2006.01)*

(86) Numéro de dépôt international:
**PCT/FR2009/000456**

(87) Numéro de publication internationale:
**WO 2009/133314 (05.11.2009 Gazette 2009/45)**

(54) **DERIVES DE 1,3-DIHYDRO-2H-PYRROLO(3,2-b) PYRIDIN-2-ONE, LEUR PREPARATION ET LEURS APPLICATIONS EN THERAPEUTIQUE**

1,3-DIHYDRO-2H-PYRROLO[3.2-B]PYRIDIN-2-ONDERIVATE, DEREN HERSTELLUNG UND DEREN THERAPEUTISCHE VERWENDUNGEN

1,3-DIHYDRO-2H-PYRROLO[3,2-B]PYRIDIN-2-ONE DERIVATIVES, PREPARATION THEREOF AND THERAPEUTIC USES THEREOF

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(30) Priorité: **21.04.2008 FR 0802195**

(43) Date de publication de la demande:
**16.03.2011 Bulletin 2011/11**

(73) Titulaire: **SANOFI**
**75013 Paris (FR)**

(72) Inventeurs:
- **COUTURIER Cédric**
  **F-75013 PARIS (FR)**
- **FOULON Loïc**
  **F-75013 PARIS (FR)**
- **SERRADEIL-LEGAL Claudine**
  **F-75013 PARIS (FR)**
- **VALETTE Gérard**
  **F-75013 PARIS - FRANCE (FR)**

(74) Mandataire: **Werner, Alain Henri et al**
**Sanofi-Aventis**
**Département Brevets**
**174, avenue de France**
**75013 Paris (FR)**

(56) Documents cités:
**EP-A- 0 636 608** **WO-A-93/15051**
**WO-A-95/18105** **WO-A-03/008407**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 294 068 B1

Printed by Jouve, 75001 PARIS (FR)

## Description

La présente invention se rapporte à de nouveaux dérivés de 1,3-dihydro-2H pyrrolo[3,2-*b*]pyridin-2-one, à leur préparation et à leur application en thérapeutique.

Les composés selon la présente invention présentent une forte affinité et une grande sélectivité pour les récepteurs $V_{1a}$ humains ou, pour certains composés, à la fois pour les récepteurs $V_{1a}$ et $V_{1b}$ humains de l'arginine-vasopressine (AVP).

L'AVP est une hormone connue pour son effet antidiurétique et son effet dans la régulation de la pression artérielle. Elle stimule plusieurs types de récepteurs : $V_1$ ($V_{1a}$,$V_{1b}$), $V_2$. Ces récepteurs sont localisés en particulier dans le foie, les vaisseaux (coronaires, rénaux, cérébraux), les plaquettes, le rein, l'utérus, les glandes surrénales, le pancréas, le système nerveux central, l'hypophyse. L'AVP exerce ainsi des effets cardiovasculaires, hépatiques, pancréatiques, antidiurétiques, agrégants des plaquettes et des effets sur le système nerveux central et périphérique, et sur la sphère utérine.

La localisation des différents récepteurs est décrite dans : S. JARD et al., Vasopressin and oxytocin receptors : an overview, in Progress in Endocrinology. H. IMURA and K. SHIZURNE ed., Experta Medica, Amsterdam, 1988, 1183-1188, ainsi que dans les articles suivants : J. Lab. Clin. Med., 1989, 114 (6), 617-632 et Pharmacol. Rev., 1991, 43 (1), 73-108.

Plus particulièrement, les récepteurs $V_{1a}$ de l'AVP sont localisés dans de nombreux organes périphériques et dans le cerveau. Ils ont été clonés chez le rat et l'homme et ils régulent la majorité des effets connus de l'AVP : l'agrégation plaquettaire ; les contractions utérines ; la contraction des vaisseaux ; la contraction des cellules mésangiales rénales, la sécrétion d'aldostérone, de cortisol, du CRF (de l'anglais corticotropin-releasing factor) et de l'hormone adrénocorticotrophique (ACTH, de l'anglais adrenocorticotrophic hormone) ; la glycogénolyse hépatique, la prolifération cellulaire et les principaux effets centraux de l'AVP (hypothermie, mémoire, anxiété, affiliation ...).

Le cortex surrénalien est aussi riche en récepteurs $V_{1a}$ impliqués dans la production de gluco- et minéralo-corticoïdes (aldostérone et cortisol). Via ces récepteurs, l'AVP (circulante ou synthétisée localement) peut induire une production d'aldostérone avec une efficacité comparable à celle de l'angiotensine II (G. GUILLON et al., Endocrinology, 1995, 136 (3), 1285-1295). Le cortisol est un puissant régulateur de la production d'ACTH, l'hormone du stress.

De récents travaux ont aussi montré que les surrénales étaient capables de libérer directement du CRF et/ou de l'ACTH via l'activation des récepteurs $V_{1a}$ et/ou $V_{1b}$ portés par les cellules de la médulla (G. MAZZOCCHI et al., Peptides, 1997, 18 (2), 191-195 ; E. GRAZZINI et al., J. Clin. Endocrinol. Metab., 1999, 84 (6), 2195-2203).

Les récepteurs $V_{1a}$ sont aussi un marqueur plus spécifique des cancers pulmonaires à petites cellules (SCLC) (P.J. WOLL et al., Biochem. Biophys. Res. Commun., 1989, 164 (1), 66-73). Ainsi, les composés selon la présente invention sont des outils de diagnostic évidents et offrent une approche thérapeutique nouvelle pour contrôler la prolifération de ces tumeurs et leur détection, à un stade même précoce (radiomarquage ; SPECT, de l'anglais Single Photon Emission Computed Tomography ; PET Scan, de l'anglais Positron Emission Tomography Scanner).

Les récepteurs $V_{1b}$ ont été initialement identifiés dans l'adénohypophyse de différentes espèces animales (rat, porc, boeuf, mouton...) y compris chez l'homme (S. Jard et al., Mol. Pharmacol., 1986, 30, 171-177 ; Y. Arsenijevic et al., J. Endocrinol., 1994, 141, 383-391 ; J. Schwartz et al., Endocrinology, 1991, 129 (2), 1107-1109 ; Y. de Keyser et al., FEBS Letters, 1994, 356, 215-220) où ils stimulent la libération d'hormone adrénocorticotrophique par l'AVP et potentialisent les effets du CRF sur la libération d'ACTH (G. E. Gdjjes et al., Nature, 1982, 299, 355). Dans l'hypothalamus, les récepteurs $V_{1b}$ induisent aussi une libération directe de CRF (Neuroendocrinology, 1994, 60, 503-508) et sont, à ces divers titres, impliqués dans les situations de stress.

Ces récepteurs $V_{1b}$ ont été clonés chez le rat, l'homme et la souris (Y. de Keyser, FEBS Letters, 1994, 356, 215-220 ; T. Sugimoto et al., J. Biol. Chem., 1994, 269 (43), 27088-27092 ; M. Saito et al., Biochem. Biophys. Res. Commun., T995,212 (3), 751-757 ; S. J. Lolait et al., Neurobiology, 1996, 92, 6783-6787 ; M. A. Ventura et al., Journal of Molecular Endocrinology, 1999, 22, 251-260) et différentes études (hybridation in situ, PCR, de l'anglais Polymerase Chain Reaction...) révèlent une localisation ubiquitaire de ces récepteurs dans divers tissus centraux (cerveau, hypothalamus et adénohypophyse, en particulier) et périphériques (rein, pancréas, surrénales, coeur, poumons, intestin, estomac, foie, mésentère, vessie, thymus, rate, utérus, rétine, thyroïde...) et dans certaines tumeurs (hypophysaires, pulmonaires...) suggérant un rôle biologique et/ou pathologique étendu de ces récepteurs et une implication potentielle dans diverses maladies.

A titre d'exemples, chez le rat, des travaux ont montré que l'AVP via les récepteurs $V_{1b}$ régule le pancréas endocrine en stimulant la sécrétion d'insuline et de glucagon (B. Lee et al., Am. J. Physiol. 269 (Endocrinol. Metab. 32) : E1095-E1100, 1995) ou la production de catécholamines dans la médullo-surrénale qui est le siège d'une synthèse locale d'AVP (E. Grazzini et al., Endocrinology, 1996, 137 (a), 3906-3914). Ainsi, dans ce dernier tissu, l'AVP via ces récepteurs aurait un rôle crucial dans certains types de phéochromocytomes surrénaliens sécrétant de l'AVP et induisant de ce fait une production soutenue de catécholamines à l'origine d'hypertension résistantes aux antagonistes des récepteurs de l'angiotensine II et aux inhibiteurs de l'enzyme de conversion.

Les récepteurs $V_{1b}$ sont aussi considérés comme un marqueur des tumeurs sécrétantes d'ACTH que sont certaines tumeurs pituitaires, certains carcinomes bronchiques (cancers pulmonaires à petites cellules ou SCLC, de l'anglais Small Cell Lung Cancers), pancréatiques, surrénaliens et thyroïdiens, induisant un syndrome de Cushing dans certains cas (J. Bertherat et al., Eur. J. Endocrinol., 1996, 135, 173 ; G. A. Wuinert et al., Lancet, 1990, 335, 991-994 ; G. Dickstein et al., J. Clin. Endocrinol. Metab., 1996, 81 (8), 2934-2941).

La présence abondante du messager des récepteurs $V_{1b}$ au niveau stomacal et intestinal, suggère une implication de l'AVP via ce récepteur sur la libération d'hormones gastro-intestinales comme la cholécystokinine, la gastrine ou la sécrétine (T. Sugimoto et al., Molecular cloning and functional expression of V1b receptor gene, dans Neurohypophysis : Recent Progress of Vasopressin and Oxytocin Research ; T. Saito, K. Kurosawa et S. Yoshida, ed., Elsevier Science, 1995, 409413).

Des dérivés de 1,3-dihydro-2H-indol-2-one ont été décrits dans certaines demandes de brevets comme des ligands des récepteurs de l'arginine-vasopressine et/ou de l'ocytocine : on peut citer les demandes de brevets WO 93/15 051, EP 636 608, EP 636 609, WO 97/15 556, WO 98/25 901, WO 01/55 130, WO 01/55 134, WO 01/64 668, WO 01/98 295, WO 03/008 407, WO 06/080 574, WO 08/025 735.

La demande internationale WO 95/18 105 concerne des composés de formule :

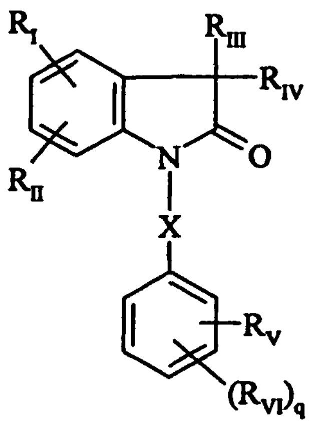

(A)

dans laquelle notamment :

- X représente $SO_2$ ;
- $R_I$, $R_{II}$, $R_{III}$, $R_{IV}$, $R_V$, $R_{VI}$ et q ont différentes valeurs.

Les composés de formule (A) ont une affinité pour les récepteurs en général de la vasopressine et/ou de l'ocytocine. En outre, cette demande ne décrit aucun exemple dans lequel la position -4- du cycle indol-2-one est occupée par un atome d'azote et $R_{IV}$ est toujours lié en position -3- du cycle indol-2-one par un atome d'azote.

En particulier le 3-[4-[[5,6-dichloro-3-(2-chlorophényl)-2-oxo-3-[(2-pipéridin-4-yléthyl)amino]-2,3-dihydro-1*H*-indol-1-yl]sulfonyl]phényl]-1,1-diéthylurée (composé $\alpha$) est décrit à l'exemple 220 et le 3-[4-[[5-chloro-3-(2-chlorophényl)-6-méthyl-2-oxo-3-[(2-pipéridin-4-yléthyl)amino]-2,3-dilxydro-1*H*-indol-1-yl]sulfonyl]phényl]-1-,1-diéthylurée (composé $\beta$) est décrit à l'exemple 277 de WO 95/018 105.

Le composé $\alpha$ présente une bonne affinité pour les récepteurs $V_{1a}$ humains de l'AVP, mais aussi pour les récepteurs $V_2$ humains de l'AVP et les récepteurs de l'ocytocine ; il n'est donc pas sélectif des récepteurs $V_{1a}$. humains de l'AVP ainsi que des récepteurs $V_{1b}$ humains de l'AVP.

Le composé $\beta$ présente une bonne affinité pour les récepteurs $V_{1a}$ humains de l'AVP, mais aussi pour les récepteurs $V_2$ humains de l'AVP ; il n'est donc pas sélectifs des récepteurs $V_{1a}$ humains de l'AVP ainsi que des récepteurs $V_{1b}$ humains de l'AVP.

On a maintenant trouvé de nouveaux composés qui présentent une forte affinité et une grande sélectivité pour les récepteurs $V_{1a}$ humains de l'AVP et qui sont des antagonistes desdits récepteurs et certains composés présentent en plus une forte affinité pour les récepteurs $V_{1b}$ humains de l'AVP.

L'invention a pour objet des composés répondant à la formule (I)

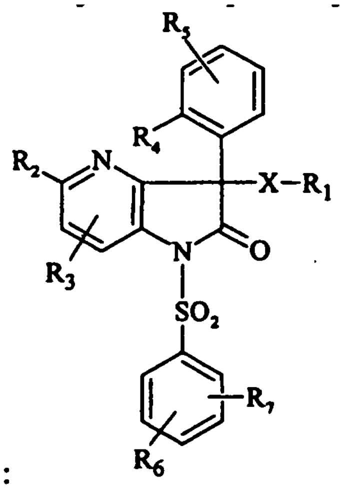

(I)

dans laquelle :

- X représente un radical bivalent $(C_1-C_5)$alkylène non substitué ou substitué une ou plusieurs fois sur un atome de carbone par un atome de fluor ou par un $(C_1-C_3)$alkyle:
- $R_1$ représente :

  un groupe $-NR_8R_9$;
  un radical pipéridin-3-yle ou pipéridin-4-yle non substitué ou substitué une ou plusieurs fois par un $(C_1-C_4)$ alkyle, un $(C_3-C_5)$cycloalkyle, les atomes de carbone pouvant être également substitués par un ou plusieurs atomes de fluor ;

- $R_2$ représente un atome d'hydrogène, un atome d'halogène, un groupe Alk, un groupe OAlk;
- $R_3$ représente un atome d'hydrogène, un atome d'halogène, un groupe Alk, un groupe OAlk ;
- $R_4$ représente un atome d'hydrogène, un atome d'halogène, un groupe Alk, un hydroxy, un groupe OAlk ;
- $R_5$ représente un atome d'hydrogène, un atome d'halogène, un groupe Alk, un hydroxy, un groupe OAlk ;
- $R_6$ représente un atome d'hydrogène, un atome d'halogène, un groupe Alk, un hydroxy, un groupe OAlk ;
- $R_7$ représente un atome d'hydrogène, un atome d'halogène, un groupe Alk, un hydroxy, un groupe OAlk ;
- $R_8$ et $R_9$ représentent chacun indépendamment un atome d'hydrogène ou un $(C_1-C_4)$alkyle ;
- ou bien $R_8$ et $R_9$ ensemble avec l'atome d'azote auquel ils sont liés constituent un radical hétérocyclique choisi parmi : l'azétidin-1-yle, la pyrrolidin-1-yle, la pipéridin-1-yle, la morpholin-4-yle, la thiomorpholin-4-yle, la pipérazin-1-yle ou la perhydroazépin-1-yle, ledit radical hétérocyclique étant non substitué ou substitué une ou plusieurs fois par un amino, un hydroxy, un $(C_1-C_4)$alkyle, un $(C_3-C_5)$cycloalkyle, un $(C_1-C_4)$alcoxy, les atomes de carbone pouvant être également substitués par un ou plusieurs atomes de fluor ;
- Alk représente un $(C_1-C_4)$alkyle non substitué ou substitué une ou plusieurs fois par un atome de fluor.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.
Les composés de formule (I) peuvent exister à l'état de bases ou de sels d'addition à des acides. De tels sels d'addition font partie de l'invention.
Ces sels sont avantageusement préparés avec des acides pharmaceutiquement acceptables mais les sels d'autres acides utiles pour la purification ou l'isolement des composés de formule (I) font également partie de l'invention.
Selon la présente invention, les N-oxydes des composés comportant une amine ou un atome d'azote font également partie de l'invention.
Par atome d'halogène on entend un atome de brome, de chlore, de fluor ou d'iode.
Par $(C_1-C_3)$alkyle ou respectivement $(C_1-C_4)$alkyle, on entend un radical alkyle linéaire ou ramifié de un à trois atomes de carbone ou respectivement de un à quatre atomes de carbone, tel que le radical méthyle, éthyle, propyle, isopropyle, butyle, isobutyle, *sec*-butyle, *tert*-butyle.
Par $(C_1-C_5)$alkylène on entend un radical bivalent de un à cinq atomes de carbone tel que le radical méthylène, éthylène, triméthylène, tétraméthylène ou le radical pentaméthylène.
Par $(C_3-C_5)$cycloalkyle on entend un radical cyclopropyle, cyclobutyle ou cyclopentyle.

Par ($C_1$-$C_4$)alcoxy on entend un radical alcoxy linéaire ou ramifié de un à quatre atomes de carbone tel que le radical méthoxy, éthoxy, propoxy, isopropoxy, butoxy, *sec*-butoxy, *tert*-butoxy.

Selon la présente invention, on préfère les composés de formule (I) dans laquelle :

- X représente un radical bivalent ($C_3$-$C_4$) alkylène non substitué ou substitué une ou plusieurs fois sur un atome de carbone par un atome de fluor ou par un ($C_{1-}C_3$) alkyle;
- $R_1$ représente :
  - un groupe -$NR_8R_9$ ;
  - un radical pipéridin-4-yle ;
- $R_2$ représente un groupe Alk, un groupe O Alk
- $R_3$ représente un atome d'hydrogène ;
- $R_4$ représente un atome d'halogène ;
- $R_5$ représente un atome d'hydrogène ;
- $R_6$ représente un atome d'halogène, un groupe OAlk ;
- $R_7$ représente un atome d'hydrogène, un groupe Alk, un groupe OAlk ;
- $R_8$ et $R_9$ ensemble avec l'atome d'azote auquel ils sont liés constituent un radical hétérocyclique choisi parmi : la pipéridin-1-yle, la pipérazin-1-yle, ledit radical hétérocyclique étant non substitué ou substitué une ou deux fois par un atome de fluor, un amino, un hydroxy, un méthyle ;
- Alk représente un ($C_1$-$C_4$)alkyle non substitué ou substitué une ou plusieurs fois par un atome de fluor ;
  à l'état de base ou de sel d'addition à un acide.

Particulièrement, on préfère les composés de formule (I) dans laquelle :

- X représente un radical bivalent triméthylène ou tétraméthylène ;
- $R_1$ représente :
  - un pipéridin-1-yle, un pipérazin-1-yle, un 4-méthylpipérazin-1-yle ;
  - un pipéridin-4-yle ;
- $R_2$ représente un méthyle, un méthoxy ;
- $R_3$ représente un atome d'hydrogène ;
- $R_4$ représente un atome de fluor ;
- $R_5$ représente un atome d'hydrogène ;
- $R_6$ représente un atome de chlore, un méthoxy, un isopropoxy, un difluorométhoxy ;
- $R_7$ représente un atome d'hydrogène, un méthyle, un méthoxy ;
  à l'état de base ou de sel d'addition à un acide.

Parmi les composés de formule (I) objets de l'invention, on peut notamment citer les composés suivants :

- 3-(2-Fluorophényl)-1-[(4-isopropoxyphényl)sulfonyl-5-méthoxy-3-(3-pipérazin-1-ylpropyl)-1,3-dihydro-2*H*-pyrrolo [3,2-*b*]pyridin-2-one ;
- 3-(2-Fluorophényl)-1-[(4-isopropoxyphényl)sulfonyl]-5-méthoxy-3-(3-pipérazin-1-ylpropyl)-1,3-dihydro-2*H*-pyrrolo [3,2-*b*]pyridin-2-one ;
- 3-(2-Fluorophényl)-5-méthoxy-1-[(3-méthoxyphényl)sulfonyl]-3-(3-pipérazin-1-ylpropyl)-1,3-dihydro-2*H*-pyrrolo [3,2-*b*]pyridin-2-one ;
- 1-[(3-Chlorophényl)sulfonyl]-3-(2-fluorophényl)-5-méthoxy-3-(3-pipérazin-1-ylpropyl)-1-,3-dihydro-2*H*-pyrrolo [3,2-*b*]pyridin-2-one ;
- 3-(2-Fluorophényl)-1-[(4-isopropoxyphényl)sulfonyl]-5-méthoxy-3-(3-pipéridin-1-ylpropyl)-1,3-dihydro-2*H*-pyrrolo [3,2-*b*]pyridin-2-one ;
- 3-(2-Fluorophényl)-1-[(4-isopropoxyphényl)sulfonyl]-5-méthoxy-3-(3-pipéridin-1-ylpropyl)-1,3-dihydro-2*H*-pyrrolo [3,2-*b*]pyridin-2-one ;
- 3-(2-Fluorophényl)-5-méthoxy-1-[(3-méthoxyphényl)sulfonyl]-3-(3-pipéridin-1-ylpropyl)-1,3-dihydro-2H-pyrrolo [3,2-*b*]pyridin-2-one, isomère ;
- 1-{[4-(Difluorométhoxy)phényl]sulfonyl}-3-(2-fluorophényl)-5-méthoxy-3-(3-pipéridin-1-ylpropyl)-1,3-dihydro-2*H*-pyrrolo[3,2-*b*]pyridin-2-one ;
- 1-[(3-Chlorophényl)sulfonyl]-3-(2-fluorophényl)-5-méthoxy-3-(3-pipéridin-1-ylpropyl)-1,3-dihydro-2*H*-pyrrolo[3,2-*b*]

pyridin-2-one ;

- 3-(2-Fluorophényl)-1-[(4-isopropoxyphényl)sulfonyl]-5-méthoxy-3-(4-pipérazin-1-ylbutyl)-1,3-dihydro-2*H*-pyrrolo [3,2-b]pyridin-2-one ;
- 3-(2-Fluorophényl)-1-[(4-isopropoxyphényl)sulfonyl]-5-méthoxy-3-[3-(4-méthylpipérazin-1-yl)propyl]-1,3-dihydro-2*H*-pyrrolo[3,2-*b*]pyridin-2-one;
- 3-(2-Fluorophényl)-1-[(4-isopropoxyphényl)sulfonyl]-5-méthoxy-3-[4-(4-méthylpipérazin-1-yl)butyl]-1,3-dihydro-2*H* pyrrolo[3,2-*b*]pyridin-2-one ;
- 3-(2-Fluorophényl)-1-[(4-isopropoxyphényl)sulfonyl]-5-méthoxy-3-(3-pipéridin-4-ylpropyl)-1,3-dihydro-2*H*-pyrrolo [3,2-b]pyridin-2-one ;
- 3-(2-Fluorophényl)-1-[(4-isopropoxyphényl)sulfonyl]-5-méthoxy-3-(4-pipérazin-1-ylbutyl)-1,3-dihydro-2*H*-pyrrolo [3,2-b]pyridin-2-one ;
- 3-(2-Fluorophényl)-1-[(4-isopropoxyphényl)sulfonyl]-5-méthyl-3-(3-pipérazin-1-ylpropyl)-1,3-dihydro-2*H*-pyrrolo [3,2-b]pyridin-2-one ;
- 3-(2-Fluorophényl)-5-méthoxy-1-[(4-méthoxy-3-méthylphényl)sulfonyl]-3-(4-pipérazin-1-ylbutyl)-1,3-dihydro-2*H*-pyrrolo[3,2-*b*]pyridin-2-one ;
- 1-[(2,4-Diméthoxyphényl)sulfonyl]-3-(2-fluorophényl)-5-méthyl-3-(3-pipérazin-1-ylpropyl)-1,3-dihydro-2*H*-pyrrolo [3,2-b]pyridin-2-one ;
- 3-(2-Fluorophényl)-5-méthoxy-1-[(4-méthoxy-3-méthylphényl)sulfonyl]-3-(3-pipérazin-1-ylpropyl)-1,3-dihydro-2*H*-pyrrolo[3,2-*b*]pyridin-2-one ;
- 1-[(2,4-Diméthoxyphényl)sulfonyl]-3-(2-fluorophényl)-5-méthoxy-3-(3-pipérazin-1-ylpropyl)-1,3-dihydro-2*H*-pyrrolo [3,2-*b*]pyridin-2-one ;
- 3-(2-Fluorophényl)-1-[(4-méthoxy-3-méthylphényl)sulfonyl]-5-méthyl-3-(3-pipérazin-1-ylpropyl)-1-,3-dihydro-2*H*-pyrrolo[3,2-*b*]pyridin-2-one ;
- 1-[(2,4-Diméthoxyphényl)sulfonyl]-3-(2-fluorophényl)-5-méthoxy-3-(4-pipérazin-1-ylbutyl)-1-,3-dilxydro-2*H*-pyrrolo [3,2-*b*]pyridin-2-one ;
- 3-(2-Fluorophényl)-1-[(4-isopropoxyphényl)sulfonyl]-5-méthyl-3-[3-(4-méthylpipérazin-1-yl)propyl]-1-,3-dihydro-2*H*-pyrrolo[3,2-*b*]pyridin-2-one ;
- 3-(2-Fluorophényl)-5-méthoxy-1-[(4-méthoxy-3-méthylphényl)sulfonyl]-3-[4-(4-méthylpipérazin-1-yl)butyl)-1,3-dihydro-2*H*-pyrrolo[3,2-b]pyridin-2-one ;

à l'état de base ou de sels d'addition à des acides, sous forme d'énantiomères purs ou de mélange racémique.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Group in Organic Synthesis », Green et al., 4th Edition, John Wiley & Sons, Inc., New York, 2007.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advanced Organic Chemistry », M. B. Smith and J. March, 6th Edition, Wiley Interscience, 2007, p.496-501.

Conformément à l'invention, on peut préparer les composés de formule (I) selon un procédé qui est caractérisé en ce que :

On fait réagir, en présence d'une base, un composé de formule :

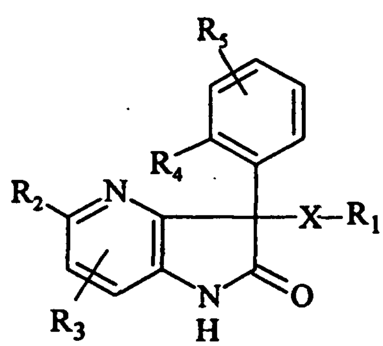

(II)

dans laquelle X, $R_1$, $R_2$, $R_3$, $R_4$ et $R_5$ sont tels que définis pour un composé de formule (I), avec un halogénure de sulfonyle de formule :

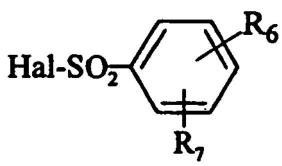

(III)

dans laquelle $R_6$ et $R_7$ sont tels que définis pour un composé de formule (I) et Hal représente un atome d'halogène.

Eventuellement, on transforme le composé de formule (I) en l'un de ses sels avec des acides minéraux ou organiques.

La réaction s'effectue en présence d'une base forte comme, par exemple, un hydrure métallique tel que l'hydrure de sodium ou un alcoolate alcalin tel que le *tert*-butylate de potassium, dans un solvant anhydre tel que le N,N-diméthylformamide ou le tétrahydrofurane et à une température comprise entre -70°C et +60°C. La réaction s'effectue de préférence en utilisant un composé de formule (III) dans laquelle Hal = Cl.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Les composés de formule (II) se préparent par réaction d'un composé 1,3-dihydro-2*H*-pyrrolo[3,2-*b*]pyridin-2-one de formule :

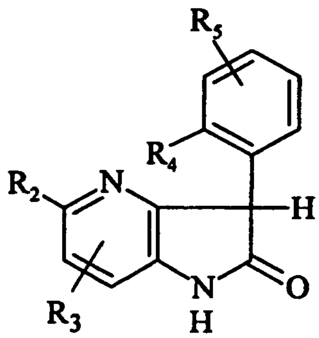

(IV)

dans laquelle $R_2$, $R_3$, $R_4$ et $R_5$ sont tels que définis pour un composé de formule (I) avec un composé de formule :

$Z\text{-}X\text{-}R_1$ (V)

dans laquelle X et $R_1$ sont tels que définis pour un composé de formule (I) et Z représente un groupe partant tel qu'un atome d'halogène, de préférence l'iode ou le brome, ou un groupe méthanesulfonate ou p-toluène sulfonate.

La réaction s'effectue en présence d'une base forte comme, par exemple, un alcoolate alcalin tel que le *tert*-butylate de potassium dans un solvant tel que le tétrahydrofurane ou le N,N-diméthylformamide et à une température comprise entre - 50°C et la température ambiante.

On peut également préparer les composés de formule (II) par réaction d'un composé de formule :

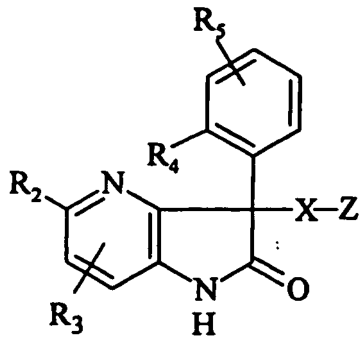

(VI)

dans laquelle X, $R_2$, $R_3$, $R_4$ et $R_5$ sont tels que définis pour un composé de formule (I) et Z représente un groupe partant tel que précédemment décrit avec un composé de formule $R_1H$ (VII). La réaction s'effectue en présence d'un carbonate de métal alcalin tel que, par exemple, le carbonate de sodium et en présence d'un halogénure de métal alcalin tel que, par exemple, l'iodure de sodium, dans un solvant tel que l'acétonitrile ou le N,N-diméthylformamide et à une température comprise entre la température ambiante et la température de reflux du solvant.
Les composés de formule (III) sont commerciaux, connus ou préparés par des méthodes connues telles que celles décrites dans EP 0 469 984 B et WO 95/18 105. Par exemple, les composés de formule (III).peuvent être préparés par halogénation des acides benzènesulfoniques correspondants ou de leurs sels, par exemple de leurs sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant tel qu'un hydrocarbure halogéné ou le N,N-diméthylformamide et à une température comprise entre -10˚C et 200˚C.

On peut également préparer les composés de formule (III) par réaction de l'acide chlorosulfonique avec un composé de formule :

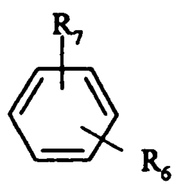

(VIII)

dans laquelle $R_6$ et $R_7$ sont tels que définis pour un composé de formule (I). La réaction s'effectue selon les modes opératoires décrits dans Chlorosulfonic Acid ; R J. Cremlyn ; The Royal Society of Chemistry, 2002.

Les composés de formule (IV) sont connus et se préparent selon des méthodes connues telles que celles décrites dans WO 95/18 105 ou dans WO 01/55 130.

On peut également préparer les composés de formule (IV) selon le SCHEMA 1 ci-après dans lequel R représente un ($C_1$-$C_4$)alkyle.

SCHEMA 1

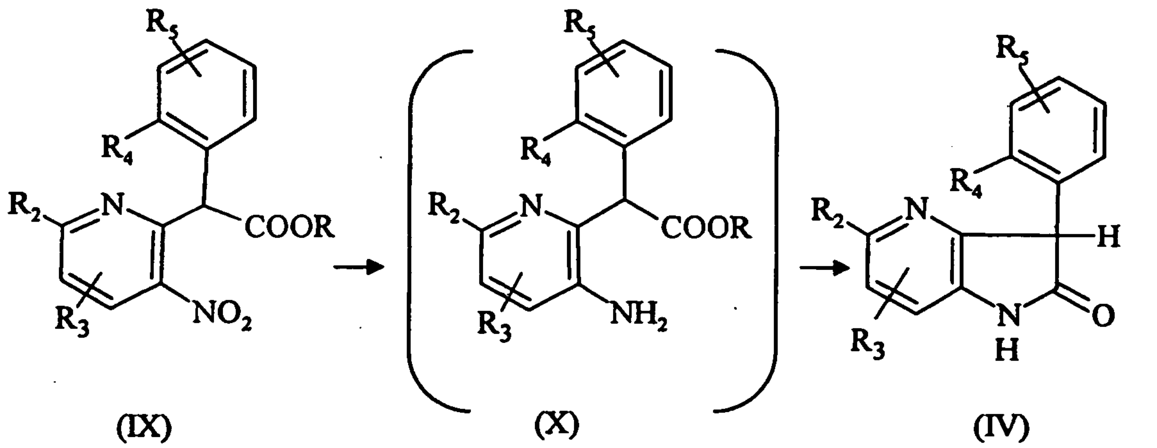

(IX) (X) (IV)

Selon le SCHEMA 1, on obtient les composés de formule (IV) par cyclisation de l'amine de formule (X) générée *in situ* par réduction du groupe nitro des composés de formule (IX). La réaction s'effectue en présence d'un métal tel que, par exemple, l'étain ou le fer en milieu acide tel que l'acide acétique, dans un solvant tel que le méthanol et à une température comprise entre la température ambiante et 100°C.

Les composés de formule (V) sont disponibles dans le commerce ou préparés selon des méthodes connues.

Les composés de formule (VI) se préparent par réaction d'un composé de formule :

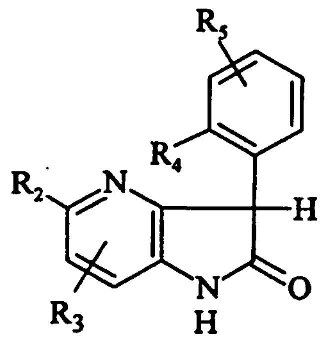

(IV)

dans laquelle $R_2$, $R_3$, $R_4$ et $R_5$ sont tels que définis pour un composé de formule (I) avec un composé de formule :

Hal-X-Z (XI)

dans laquelle X, est tel que défini pour un composé de formule (I), Z est tel que défini précédemment et Hal représente un atome d'halogène.

La réaction s'effectue en présence d'une base forte comme, par exemple, un alcoolate alcalin tel que le *tert*-butylate de potassium dans un solvant tel que le tétrahydrofurane ou le N,N-diméthylformamide et à une température comprise entre - 50°C et la température ambiante.

Les composés de formule (VI) se préparent également par cyclisation d'un composé de formule :

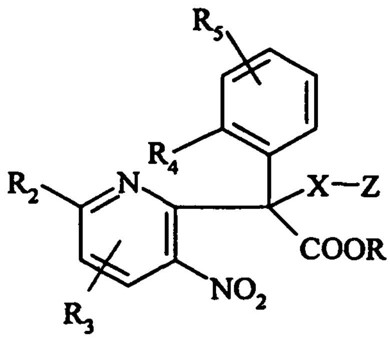

(XII)

dans laquelle $R_2$, $R_3$, $R_4$, $R_5$, X et Z sont tels que définis pour un composé de formule (I).

La réaction s'effectue en présence d'un métal tel que, par exemple, l'étain ou le fer en milieu acide tel que l'acide acétique, dans un solvant tel que le méthanol et à une température comprise entre la température ambiante et 100°C.

Les composés de formule (VII) sont commerciaux, connus ou préparés selon des méthodes connues de l'homme de l'art.

Les composés de formule (VIII) sont connus ou se préparent selon des méthodes connues.

Les composés de formule (IX) se préparent par réaction des composés de formule:

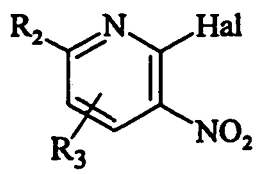

(XIII)

dans laquelle $R_2$ et $R_3$ sont tels que définis pour un composé de formule (I) et Hal représente un atome d'halogène, avec un composé de formule :

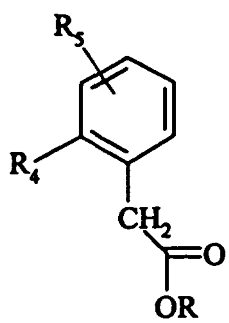

(XIV)

dans laquelle $R_4$ et $R_5$ sont tels que définis pour un composé de formule (I).

La réaction s'effectue en présence d'une base forte comme, par exemple, un alcoolate alcalin tel que le *tert*-butylate de potassium ou comme un hydrure métallique tel que l'hydrure de sodium, dans un solvant anhydre tel que le N,N-diméthylformamide et à une température comprise entre -50°C et la température ambiante.

Les composés de formule (XI), (XIII) et (XIV) sont préparés selon des méthodes bien connues de l'homme du métier.

Les composés de formule (XII) se préparent par réaction d'un composé de formule :

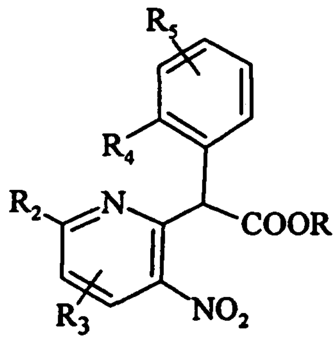

(IX)

dans laquelle $R_2$, $R_3$, $R_4$ et $R_5$ sont tels que définis pour un composé de formule (I) et R est un groupe $C_1$-$C_4$ alkyle avec un composé de formule :

Hal-X-Z (XI)

dans laquelle X, est tel que défini pour un composé de formule (I), Z est tel que défini précédemment et Hal représente un atome d'halogène.

La réaction s'effectue en présence d'une base forte comme un alcoolate alcalin tel que le *tert*-butylate de potassium dans un solvant tel que le tétrahydrofurane ou le N,N-diméthylformamide et à une température comprise entre -50°C et la température ambiante.

Les N-oxydes des composés comportant une amine ou un atome d'azote sont préparés selon les méthodes connues de l'homme du métier par réaction de l'amine avec des peracides organiques tels que les acides peracétique, trifluoroperacétiques, performique, perbenzoïques ou ses dérivés tel l'acide 3-chloroperbenzoïque, à des températures comprises entre 0°C et 90°C, de préférence à des températures inférieures à 50°C.

Pour obtenir les composés de formule (I) sous forme d'isomères optiquement purs, on peut utiliser les techniques classiques de séparation : par exemple des recristallisations fractionnées d'un sel formé à partir d'une base racémique avec un acide optiquement actif dont le principe est bien connu ou les techniques classiques de chromatographies préparatives en phase liquide ou en milieu supercritique sur phases chirales.

On peut également préparer les composés de formule (I) optiquement purs à partir d'un composé intermédiaire optiquement pur utile pour la préparation des composés de formule (I) selon les techniques décrites dans WO 03/008 407.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans les TABLEAUX (I, II et III) ci-après, qui illustrent les structures chimiques et les propriétés physiques de quelques composés selon l'invention.

Dans les Préparations et dans les Exemples on utilise les abréviations suivantes :

EtOH : éthanol
éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
méthanol : MeOH
éther chlorhydrique 2N : solution 2N d'acide chlorhydrique dans l'éther diéthylique
F : point de fusion
TA : température ambiante
CLHP : chromatographie liquide haute performance
silice H : gel de silice 60 H commercialisé par Merck (DARMSTAD)
solution tampon pH = 2 : solution de 16,66 g de $KHSO_4$ et 32,32 g de $K_2SO_4$ dans 1 litre d'eau.

Les spectres de résonance magnétique nucléaire du proton (RMN $^1$H) sont enregistrés dans le DMSO-$d_6$. Les déplacements chimiques δ sont exprimés en parties par million (ppm). Pour l'interprétation des spectres, on utilise les abréviations suivantes : s : singulet, d : doublet, t : triplet, q : quadruplet, m : massif, mt : multiplet, se : singulet élargi,

dd : doublet dédoublé.

Les pouvoirs rotatoires sont mesurés sur un polarimètre PERKIN-ELMER 241. Les mélanges de solvants sont quantifiés en rapport volumétriques.

Les composés selon l'invention sont analysés par couplage LC/UV/MS (chromatographie liquide/détection UV/spectrométrie de masse). On mesure le pic moléculaire ($MH^+$) et le temps de rétention (tr) en minutes.

**Système 1 à pH3 : Méthode 1 (M1)**

Instrument (Agilent) : Chaine HPLC : série 1100 ;
Spectromètre de masse : MSD SL (Agilent)
Logiciel : Chemstation version B.01.03 de Agilent
LC/UV
Colonne : Symmetry C 18 3.5 µm (2,1x50mm) (Waters)
Température colonne : 25°C
Eluants : A : $H_2O$ + 0,005% TFA
B : $CH_3CN$ + 0,005% TFA
Débit : 0,4ml/min
Gradient :

| Temps (min.) | % (v/v) A/B |
|---|---|
| 0 | 100/0 |
| 10 | 0/100 |
| 15 | 0/100 |

Détection UV : 220 nm
Volume d'injection : 2 µl d'une solution à 0,5 mg/ml
MS
Mode d'ionisation : Electrospray mode positif $ESI^+$
Gamme de masse : 90-1500 uma

**Système 2 à pH7 : Méthode 2 (M2)**

Instrument (Agilent) : Chaine HPLC : série 1100 ;
Spectromètre de masse : MSD SL (Agilent)
Logiciel : Chemstation version B.01.03 de Agilent
**LC/UV**
Colonne : X Terra C18 3.5 µm (2.1x50mm) (Waters)
Température colonne : 30°C
Eluants : A : Tampon Acétate d'ammonium 10mM pH7
B : $CH_3CN$
Débit : 0,4ml/min
Gradient :

| Temps (min.) | % (v/v) A/B |
|---|---|
| 0 | 100/0 |
| 10 | 10/90 |
| 15 | 10/90 |

Détection UV : 220 nm
Volume d'injection : 2 µl d'une solution à 0,5 mg/ml
MS
Mode d'ionisation : Electrospray mode positif est
Gamme de masse : 90-1500 uma

**Système 3 à pH2.2 : Méthode 3 (M3)**

Instrument (Waters) : Chaine HPLC : Alliance 2695 ;
Détecteur UV : PDA 996
Spectromètre de masse : Platform LCZ (Micromass)
Logiciel : MassLynx version 4.0 de Waters-Micromass
LC/UV
Colonne : Symmetry C18 3.5 μm (2.1x50mm) (Waters)
Température colonne : 40°C
Eluants : A : $H_2O$ + 0,05% TFA
B : $CH_3CN$ + 0,035% TFA
Débit : 0,5ml/min
Gradient :

| Temps (min.) | % (v/v) A/B |
|---|---|
| 0,0 | 100/0 |
| 6,0 | 0/100 |
| 7,0 | 0/100 |
| 7,1 | 100/0 |
| 10,0 | 100/0 |

Détection UV : 220 nm
Volume d'injection : 2 μl d'une solution à 0,5 mg/ml
MS
Mode d'ionisation : Electrospray mode positif $ESI^+$
Gamme de masse : 120-1500 uma
**Système 4 à pH3 : Méthode 4 (M4)**
Instrument (Agilent) : Chaine HPLC : série 1100 ;
Spectromètre de masse : MSD SL (Agilent)
Logiciel : Chemstation version B.01.03 de Agilent
LC/UV
Colonne : Symmetry C18 3.5 μm (2.1x50mm) (Waters)
Température colonne : 25°C
Eluants : A : $H_2O$ + 0,005% TFA
B : $CH_3CN$ + 0,005% TFA
Débit : 0,4ml/min
Gradient :

| Temps (min.) | % (v/v) A/B |
|---|---|
| 0 | 100/0 |
| 10 | 0/100 |
| 15 | 0/100 |

Détection UV : 220 nm
Volume d'injection : 2 μl d'une solution à 0,5 mg/ml
MS
Mode d'ionisation : Electrospray mode positif $ESI^+$
Gamme de masse : 90-1500 uma

PREPARATIONS

1. Préparations des composés de formule (IX).

Préparation 1.1

(2-Fluorophényl)(6-méthoxy-3-nitropyridin-2-yl)acétate de méthyle.

$R_2$ = OMe ; $R_3$ = H ; $R_4$ = F ; $R_5$ = H ; R = Me (IX) :

A 12,7 g d'hydure de sodium à 60% dans l'huile en suspension dans 600 ml de DMF est ajouté à 0˚C, goutte à goutte, le mélange de 20 g de 2-chloro-6-méthoxy-3-nitropyridine et 20,6 g de 2-fluorophényl acétate de méthyle en solution dans 100 ml de DMF. Le mélange réactionnel est agité pendant 3 h en laissant la température remonter à TA. On ajoute 500 ml d'une solution aqueuse de $NaHCO_3$ à 5% et extrait avec 250 ml d'AcOEt. On lave la phase organique avec 300 ml d'une solution aqueuse de $NaHCO_3$ à 5%, la sèche sur $Na_2SO_4$, puis évapore les solvants sous pression réduite. Le résidu est purifié par chromatographie sur gel de silice en éluant avec un mélange cyclohexane/acétate d'éthyle pour donner le composé attendu sous forme d'un liquide.

2. Préparations des composés de formule (IV).

Préparation 2.1

3-(2-Fluorophényl)-5-méthoxy-1,3-dihydro-2*H*-pyrrolo[3,2-b]pyridin-2-one.

$R_2$ = OMe ; $R_3$ = H ; $R_4$ = F ; $R_5$ = H. (IV) :

A 37 g du composé préparé lors de la préparation 1.1, dans 530 ml d'EtOH est ajouté 29 g de fer puis 42 ml d'acide acétique. Le mélange réactionnel est agité à reflux pendant 5 h. Les solvants sont évaporés partiellement sous pression réduite puis est ajouté 250 ml d'une solution aqueuse de $NaHCO_3$ à 5% et 300 ml d'AcOEt. Après agitation, le mélange est filtré. Après décantation du filtrat, la phase organique est lavée avec 100 ml d'une solution aqueuse de $NaHCO_3$ à 5%, séchée sur $Na_2SO_4$ et concentrée sous pression réduite. Le résidu solide obtenu est repris avec de l'éther iso, filtré, séché à 50˚C sous vide.
$MH^+$ = 259 ; tr = 6,60 mn (M1)

3. Préparations des composés de formule (VI).

Préparation 3.1.

3-(3-Chloropropyl)-3-(2-fluorophényl)-5-méthoxy-1-,3-dihydro-2*H*-pyrrolo[3,2-b]pyridin-2-one, isomère dextrogyre et isomère lévogyre. (V) : $R_2$ = OMe ; $R_3$ = H ; $R_4$ = F ; $R_5$ = H ; X = $-(CH_2)_3-$ ; Z = Cl

A) 5-Chloro-2-(2-fluorophényl)-2-(6-méthoxy-3-nitropyridin-2-yl) pentanoate de méthyle (XII).

A 9,3 g d'hydure de sodium à 60% dans l'huile en suspension dans 900 ml de DMF est ajouté à 0˚C, goutte à goutte, le mélange de 20 g de 2-chloro-6-méthoxy-3-nitropyridine et 19,6 g de 2-fluorophényl acétate de méthyle en solution dans 100 ml de DMF. Le mélange réactionnel est agité pendant 3 h en laissant la température remonter à TA puis est ajouté 34 ml de 1-chloro-3-iodopropane. Le mélange est agité à TA pendant 48 h. On ajoute 500 ml d'une solution aqueuse de $NaHCO_3$ à 5%, et extrait avec 500 ml d'AcOEt. On lave la phase organique avec 300 ml d'une solution aqueuse de $NaHCO_3$ à 5%, la sèche sur $Na_2SO_4$, puis évapore les solvants sous pression réduite. Le résidu est purifié par chromatographie sur gel silice en éluant avec un mélange cyclohexane/acétate d'éthyle pour donner le composé attendu sous forme liquide qui est engagé tel quel dans l'étape suivante.

B) 3-(3-Chloropropyl)-3-(2-fluorophényl)-5-méthoxy-1,3-dihydro-2H pyrrolo[3,2-b]pyridin-2-one, isomère dextrogyre et isomère lévogyre.

A 42 g du composé de l'étape précédente, dans 530 ml d'EtOH est ajouté 29,6 g de fer puis 43 ml d'acide acétique. Le mélange réactionnel est agité à reflux pendant 5 h. Les solvants sont évaporés partiellement sous pression réduite puis est ajouté 250 ml d'une solution aqueuse de $NaHCO_3$ à 5%, et 500 ml d'AcOEt. Après agitation, le mélange réactionnel est filtré. Après décantation du filtrat, la phase organique est lavée avec 100 ml d'une solution aqueuse de $NaHCO_3$ à 5% séchée sur $Na_2SO_4$ et concentrée sous pression réduite. Le résidu solide obtenu est repris avec de l'éther iso, filtré, séché à 50°C sous vide.

Les énantionères du composé ainsi obtenu sont séparés par chromatographie liquide sur phase chirale dans les conditions suivantes:

Appareillage : Système de chromatographie Waters Delta Prep 4000 ;
Colonne chirale: CHIRALPAK AS-VCSP ;
Phase mobile : Acétonitrile 100%;
Débit : 120 ml/minute;
Pression : 50 bar ;
Détection UV : 254 nm.

Après séparation des énantiomères on obtient :

- l'isomère dextrogyre sous forme d'un solide :

$$\alpha_D^{20} = +1,2° \text{ (c = 1 ; MeOH) ;}$$

F = 65°C

- l'isomère lévogyre sous forme d'un solide :

$$\alpha_D^{20} = -1,5° \text{ (c = 1 ; MeOH) ;}$$

F = 63°C

Préparation 3.2.

3-(4-Chlorobutyl)-3-(2-fluorophényl)-5-méthoxy-1,3-dihydro-2H pyrrolo[3,2-b]pyridin-2-one (VI), isomère dextrogyre et isomère lévogyre. (VI) : $R_2$ = OMe ; $R_3$ = H ; $R_4$ = F ; $R_5$ = H ; X = $-(CH_2)_4-$ ; Z = Cl

A) 6-Chloro-2-(2-fluorophényl)-2-(6-méthoxy-3-nitropyridin-2-yl)hexanoate de méthyle (XII).

A 9,3 g d'hydure de sodium à 60% dans l'huile en suspension dans 900 ml de DMF est ajouté à 0°C, goutte à goutte, le mélange de 20 g de 2-chloro-6-méthoxy-3-nitropyridine et 19,6 g de 2-fluorophényl acétate de méthyle en solution dans 100 ml de DMF. Le mélange réactionnel est agité pendant 3 h en laissant la température remonter à température ambiante puis est ajouté 34 ml d'iodochlorobutane. Le mélange est agité à TA pendant 48 h. On ajoute 500 ml d'une solution aqueuse de $NaHCO_3$ à 5%, et extrait avec 500 ml d'AcOEt. On lave la phase organique avec 300 ml d'une solution aqueuse de $NaHCO_3$ à 5%, la sèche sur $Na_2SO_4$, puis évapore les solvants sous pression réduite. Le résidu est purifié par chromatographie sur gel de silice en éluant avec un mélange cyclohexane/acétate d'éthyle pour donner le composé attendu sous forme d'un liquide qui est engagé tel quel dans l'étape suivante.

B) 3-(4-Chlorobutyl)-3-(2-fluorophényl)-5-méthoxy-1,3-dihydro-2H pyrrolo[3,2-b]pyridin-2-one (VI).

A 42 g du composé de l'étape précédente, dans 530 ml d'éthanol est ajouté 29,6 g de fer puis 43 ml d'acide acétique. Le mélange réactionnel est agité à reflux pendant 5 h. Les solvants sont évaporés partiellement sous pression réduite puis est ajouté 250 ml d'une solution aqueuse de $NaHCO_3$ à 5%, et 500 ml d'AcOEt. Après agitation, le mélange

réactionnel est filtré. Après décantation du filtrat, la phase organique est lavée avec 100 ml d'une solution aqueuse de $NaHCO_3$ à 5%, séchée sur $Na_2SO_4$ et concentrée sous pression réduite. Le résidu solide obtenu est repris avec de l'éther iso, filtré, séché à 50°C sous vide pour donner le produit attendu sous forme racémique.

Les énantionères du composé ainsi obtenu sont séparés par chromatographie liquide sur phase chirale dans les conditions suivantes:

Appareillage : Système de chromatographie Waters Delta Prep 4000 ;
Colonne chirale: CHIRALPAK AS-VCSP ;
Phase mobile : Acétonitrile 100% ;
Débit : 120 ml/minute ;
Pression : 50 bar ;
Détection UV : 254 nm.

Après séparation des énantiomères on obtient :

- l'isomère dextrogyre sous forme d'un solide :

$$\alpha_D^{20} = +13{,}5° \ (c = 1 \ ; MeOH) \ ;$$

- l'isomère lévogyre sous forme d'un solide :

$$\alpha_D^{20} = -14{,}6 (c = 1 \ ; MeOH) \ ;$$

Préparation 3.3.

3-(3-Chloropropyl)-3-(2-fluorophényl)-5-méthyl-1,3-dihydro-2H-pyrrolo[3,2-b]pyridin-2-one.

(VI) : $R_2$ = Me ; $R_3$ = H ; $R_4$ = F ; $R_5$ = H ; X = $-(CH_2)_3-$ ; Z = Cl.

A) 5-Chloro-2-(2-fluorophényl)-2-(6-méthyl-3-nitropyridin-2-yl) pentanoate de méthyle (XII).

A 11,6 g d'hydure de sodium à 60 % dans l'huile en suspension dans 100 ml de DMF est ajouté à -10°C, goutte à goutte, le mélange de 20 g de 2-chloro-6-méthyl-3-nitropyridine et 21,5 g de 2-fluorophényl acétate de méthyle en solution dans 100 ml de DMF. Le mélange réactionnel est agité pendant 1h30 en laissant la température remonter à TA ; puis est ajouté 19,1 ml de 1-chloro-3-iodopropane. Le mélange est agité à TA pendant 18 h. Le mélange est versé sur 600 ml d'une solution aqueuse de $NH_4Cl$ à 10%, et extrait avec 300 ml d'AcOEt. On lave la phase organique à la saumure, sèche sur $MgSO_4$, filtre, puis concentre sous pression réduite. Le résidu est purifié par chromatographie sur gel silice en éluant avec un mélange heptane/dichlorométhane pour donner le composé attendu sous forme d'une huile orange.
$MH^+$ = 381 ; tr = 9,15 mn (M4)

B) 3-(3-Chloropropyl)-3-(2-fluorophényl)-5-méthyl-1,3-dihydro-2H-pyrrolo[3,2-b]pyridin-2-one (VI).

A 13,6 g du composé de l'étape précédente en solution dans 120 ml de MeOH, sont ajoutés 6 g de fer puis 14,2 ml d'acide acétique. Le mélange réactionnel est agité à reflux pendant 3 h. Le mélange est concentré sous pression réduite, puis le résidu est repris dans un mélange de 200 ml d'AcOEt et 500 ml d'acide chlorhydrique 1N. La phase organique est lavée à la saumure, séchée sur $MgSO_4$, filtrée et concentrée sous pression réduite. Le résidu solide obtenu est repris dans l'éther iso, filtré, séché à 50° C sous vide.
$MH^+$ = 319 ; tr = 7,10 mn (M4)

4. Préparations des composés de formule (II).

Préparation 4.1.

4-{3-[3-(2-Fluorophényl)-5-méthoxy-2-oxo-2,3-dihydro-1*H*-pyrrolo[3,2-*b*]pyridin-3-yl]propyl}piperazine-1-carboxylate de tert-butyle, isomère unique. (II) : $R_2$ = OMe ; $R_3$ = H ; $R_4$ = F ; $R_5$ = H ;

X = $-(CH_2)_3-$ ; $R_1$ = 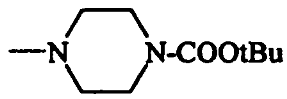

A une solution de 0,3g de composé de la préparation 3.1 isomère lévogyre dans 5 ml de DMF est ajouté 0,13g d'iodure de sodium, 0,28g de carbonate de sodium et 0,41g de pipérazine-1-carboxylate *tert*-butyle. Le mélange est agité à 85˚C pendant 6 h. On ajoute une solution aqueuse saturée de $NH_4Cl$ et extrait avec de l'AcOEt. On lave la phase organique avec une solution aqueuse saturée de NaCl, la sèche sur $Na_2SO_4$, puis évapore les solvants sous pression réduite. Le résidu est purifié par chromatographie sur gel de silice en éluant avec un mélange dichlorométhane/méthanol pour donner le composé attendu sous forme d'une huile.
$MH^+$ = 485 ; tr = 5,67 mn (M1)

Préparation 4.2

4-{3-[3-(2-Fluorophényl)-5-méthoxy-2-oxo-2,3-dihydro-1*H*-pyrrolo[3,2-*b*]pyridin-3-yl]propyl}pipérazine-1-carboxylate de *tert-butyle,* isomère unique. (II) : $R_2$ = OMe ; $R_3$ = H ; $R_4$ = F ; $R_5$ = H ;

X = $-(CH_2)_3-$ ; $R_1$ = 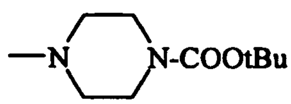

En utilisant le même mode opératoire décrit à la préparation 4.1 à partir du composé de la préparation 3.1 isomère dextrogyre on obtient le composé attendu.
$MH^+$= 485 ; tr = 5,68 mn (Méthode M1)

Préparation 4.3

3-(2-Fluorophényl)-5-méthoxy-3-(3-pipéridin-1-ylpropyl)-1,3-dihydro-2*H*-pyrrolo[3,2-*b*]pyridin-2-one, isomère unique. (II) : $R_2$ = OMe ; $R_3$ = H ; $R_4$ = F ; $R_5$ = H ;

X = $-(CH_2)_3-$ ; $R_1$ = 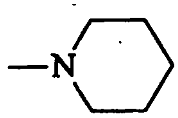

A une solution de 0,3g de composé de la préparation 3.1 isomère lévogyre dans 5 ml de DMF est ajouté 0,13g d'iodure de sodium, 0,28g de carbonate de sodium et 0,45 g de pipéridine. Le mélange est agité à 85˚C pendant 6 h. On ajoute une solution aqueuse saturée de $NH_4Cl$ et extrait avec de l'AcOEt. On lave la phase organique avec une solution aqueuse saturée de NaCl, la sèche sur $Na_2SO_4$, puis évapore les solvants sous pression réduite. Le résidu est purifié par chromatographie sur gel de silice en éluant avec un mélange dichlorométhane/méthanol pour donner le composé attendu sous forme d'une huile.
$MH^+$ = 384; tr = 5,02 mn (Méthode M1)

Préparation 4.4

3-(2-Fluorophényl)-5-méthoxy-3-(3-pipéridin-1-ylpropyl)-1,3-dihydro-2*H*-pyrrolo[3,2-*b*]pyridin-2-one, isomère unique. (II) : $R_2$ = OMe ; $R_3$ = H ; $R_4$ = F ; $R_5$ = H ;

$X = -(CH_2)_3-$ ; $R_1$ = 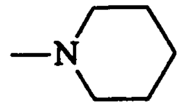

En utilisant le même mode opératoire décrit à la préparation 4.3 à partir du composé de la préparation 3.1 isomère dextrogyre on obtient le composé attendu.
$MH^+$= 384 ; tr = 5,03 mn (M1)

Préparation 4.5

4-{4-[3-(2-Fluorophényl)-5-méthoxy-2-oxo-2,3-dihydro-1*H* pyrrolo[3,2-*b*]pyridin-3-yl]butyl)pipérazine-1-carboxylate de *tert*-butyle. (II) : $R_2$ = OMe ; $R_3$ = H ; $R_4$ = F ; $R_5$ = H ;

$X = -(CH_2)_4-$ ; $R_1$ = 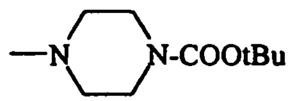

A une solution de 0,3g de composé de la préparation 3.2 dans 5 ml de DMF est ajouté 0,13 g d'iodure de sodium, 0,28 g de carbonate de sodium et 0,41 g de pipérazine-1-carboxylate *tert*-butyle. Le mélange est agité à 85°C pendant 6 h. On ajoute une solution aqueuse saturée de $NH_4Cl$ et extrait avec de l'AcOEt. On lave la phase organique avec une solution aqueuse saturée de NaCl, la sèche sur $Na_2SO_4$, puis évapore les solvants sous pression réduite. Le résidu est purifié par chromatographie sur gel de silice en éluant avec un mélange dichlorométhane/méthanol pour donner le composé attendu sous forme d'une huile.
$MH^+$ = 399 ; tr = 5,29 mn (M1)

Préparation 4.6

4-{3-[3-(2-Fluorophényl)-5-méthyl-2-oxo-2,3-dihydro-1*H*-pyrrolo[3,2-*b*]pyridin-3-yl]propyl}pipérazine-1-carboxylate de *tert*-butyle. (II) : $R_2$ = Me ; $R_3$ = H ; $R_4$ = F ; $R_5$ = H ;

$X = -(CH_2)_3-$ ; $R_1$ = 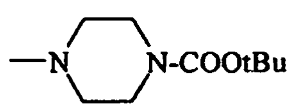

A une solution de 3,4 g de composé de la préparation 3.3 dans 40 ml de DMF, sont ajoutés 1,6 g d'iodure de sodium, 1,13 g de carbonate de sodium et 2,98 g de pipérazine-1-carboxylate de tert-butyle. Le mélange est agité à 100° C pendant 3 h. On ajoute 300 ml d'une solution aqueuse de $NH_4Cl$ à 10 % et extrait avec de l'AcOEt. On lave la phase organique avec une solution aqueuse saturée de NaCl, la sèche sur $MgSO_4$, filtre et concentre sous pression réduite. Le résidu est trituré dans l'éther iso et le composé attendu est obtenu sous forme d'un précipité orange pâle.
$MH^+$ = 469 ; tr = 5,35 mn (M4)
Les énantionères du composé ainsi obtenu sont séparés par chromatographie liquide sur phase chirale dans les conditions suivantes :

Appareillage : Système de chromatographie Waters Delta Prep 4000 ;

Colonne chirale: CHIRALPAK AS-VCSP ;
Phase mobile : Acétonitrile 100% ;
Débit : 120 ml/minute ;
Pression : 50 bar ;
Détection UV : 254 nm.
Après séparation des énantiomères on obtient :

- l'isomère dextrogyre sous forme d'une résine :

$$\alpha_D^{20} = + 15,1° \text{ (c = 1 ; MeOH) ;}$$

MH⁺ = 469 ; 4,82 mn (M4)

- l'isomère lévogyre sous forme d'une résine :

$$\alpha_D^{20} = - 12,3° \text{ (c = 1 ; MeOH) ;}$$

$MH^+$ = 469 ; 3,71 mn (M3)

Préparation 4.7

3-(2-Fluorophényl)-5-méthoxy-3-[3-(4-méthylpipérazin-1-yl)propyl]-1,3-dihydro-2*H*-pyrrolo[3,2-*b*]pyridin-2-one, isomère unique. (II) : $R_2$ = OMe ; $R_3$ = H ; $R_4$ = F ; $R_3$ = H ;

$X = -(CH_2)_3- ; R_1 =$ 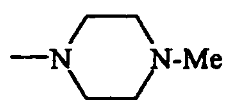

A une solution de 167 mg de composé de la préparation 3.1 isomère dextrogyre dans 2 ml de DMF, sont ajoutés 75 mg d'iodure de sodium, 53 mg de carbonate de sodium et 84 µl de 1-méthylpipérazine. Le mélange est agité à 100° C pendant 1h30. On ajoute 30 ml d'une solution aqueuse de $NH_4Cl$ à 10 % et extrait avec de l'AcOEt. On lave la phase organique avec une solution aqueuse saturée de NaCl, la sèche sur $MgSO_4$, filtre et concentre sous pression réduite. Le résidu est purifié par chromatographie sur gel silice en éluant avec un mélange dichlorométhane/MeOH pour donner le composé attendu sous forme d'une résine.
$MH^+$ = 399 ; tr = 3,32 mn (M3)

Préparation 4.8

4-{4-[3-(2-Fluorophényl)-5-méthoxy-2-oxo-2,3-dihydro-1*H*-pyrrolo[3,2-b]pyridin-3-yl]butyl}pipérazine-1-carboxylate de tert-butyle, isomère unique. (II) $R_2$ = OMe ; $R_3$ = H ; $R_4$ = F ; $R_5$ = H ;

$X = -(CH_2)_3- ; R_1 =$ 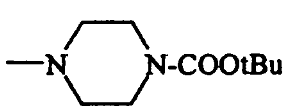

En utilisant le même mode opératoire décrit à la préparation 4.5 à partir du composé de la préparation 3.2 isomère dextrogyre, on obtient le composé attendu.
$MH^+$ = 499 ; tr = 5,32 mn (M4)

Préparation 4.9

4-{3-[3-(2-Fluorophényl)-5-méthoxy-2-oxo-2,3-dihydro-1*H*-pyrrolo[3,2-*b*]pyridin-3-yl]propyl}pipéridine-1-carboxylate de tert-butyle. (II) : $R_2$ = OMe ; $R_3$ = H ; $R_4$ = F ; $R_5$ = H ;

$X = -(CH_2)_3-$ ; $R_1$ = 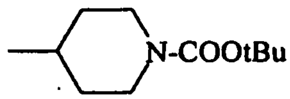

A une solution de 0,6 g du composé obtenu à la préparation 2.1 dans 20 ml de DMF, sont ajoutés, à -40°C, 0,66 g de tBuOK, puis après 15 min 0,91 g de 4-(3-iodopropyl)pipéridine-1-carboxylate de *tert*-butyle. Le mélange est agité pendant 4 h après retour à -20°C, puis dilué avec 150 ml AcOEt et 300 ml d'une solution aqueuse de $NH_4Cl$ à 10 % ; la phase organique est lavée par une solution saturée de NaCl, séchée sur $MgSO_4$, filtrée et concentrée sous pression réduite. Le résidu est purifié par chromatographie sur gel silice en éluant avec un mélange heptane/AcOEt, puis par un mélange dichlorométhane/MeOH pour donner le composé attendu sous forme d'une résine.
$MH^+$ = 484 ; tr = 9,51 mn (M4)

EXEMPLE 1 : Composé N°1

Chlorhydrate de 3-(2-fluorophényl)-1-[(4-isopropoxyphényl)sulfonyl]-5-méthoxy-3-(3-pipérazin-1-ylpropyl)-1,3-dilxydro-2*H*-pyrrolo[3,2-*b*]pyridin-2-one, isomère unique.
A une solution de 0,48g de composé de la préparation 4.1 dans 8 ml de THF est ajoutée à 0°C 0,13 g de tert-butylate de potassium. Après 15 minutes d'agitation à 0°C est ajouté . 0,12 g de chlorure de 4-isopropoxybenzènesulfonyle. Après 6 heures d'agitation à 20°C, le mélange est hydrolysé par une solution saturée de chlorure d'ammonium puis extrait à l'AcOEt. La phase organique est lavée à l'eau, séchée sur $MgSO_4$, filtrée puis évaporée à sec, purifiée par chromatographie sur gel silice en éluant avec un mélange dichlorométhane/méthanol. Le produit est ensuite agité dans une solution d'éther chlorhydrique 2N et est ensuite filtré pour obtenir le composé désiré sous forme d'une poudre blanche.
F = 123°C
$MH^+$ = 583; tr = 6,42 mn (Méthode M1)

EXEMPLE 2 : Composé N°3

Chlorhydrate de 3-(2-fluorophényl)-5-méthoxy-1-[(3-methoxyphényl)sulfonyl]-3-(3-pipérazin-1-ylpropyl)-1,3-dihydro-2*H*-pyrrolo[3;2-*b*]pyridin-2-one, isomère unique.
En utilisant le même mode opératoire décrit à l'exemple 1 à partir du composé de la préparation 4.2 isomère unique et du chlorure de 3-méthoxybenzènesulfonyle on obtient le composé attendu.
F = 178°C
$MH^+$ = 555 ; tr = 5,49mn (Méthode M1)

EXEMPLE 3 : Composé N°5

Chlorhydrate de 3-(2-fluorophényl)-1-[(4-isopropoxyphényl)sulfonyl]-5-méthoxy-3-(3-pipéridin-1-ylpropyl)-1,3-dihydro-2*H*-pyrrolo[3,2-*b*]pyridin-2-one, isomère unique.
En utilisant le même mode opératoire décrit à l'exemple 1 à partir du composé de la préparation 4.3 isomère unique et du chlorure de 4-isopropoxybenzènesulfonyle on obtient le composé attendu.
F =156°C
$MH^+$ = 582; tr = 6,57 mn (Méthode M1)

EXEMPLE 4 : Composé N° 7

Chlorhydrate de 3-(2-fluorophenyl)-5-méthoxy-1-[(3-methoxyphenyl)sulfonyl]-3-(3-piperidin-1-ylpropyl)-1,3-dihydro-2*H*-pyrrolo[3,2-b]pyridin-2-one, isomère unique.
En utilisant le même mode opératoire décrit à l'exemple 1 à partir du composé de la préparation 4.4 isomère unique et du chlorure de 3-méthoxybenzènesulfonyle, on obtient le composé attendu.

F = 148°C
$MH^+$ = 554 ; tr = 6,59 mn (Méthode M1)

EXEMPLE 5 : Composé N°10

3-(2-Fluorophenyl)-1-[(4-isopropoxyphenyl)sulfonyl]-5-méthoxy-3-(4-piperazin-1-ylbutyl)-1,3-dihydro-2*H*-pyrrolo[3,2-*b*]pyridin-2-one, mélange racémique.

A une solution de 0,48g du composé racémique de la préparation 4.5 dans 8 ml de THF est ajoutée à 0°C 0,13 g de *tert*-butylate de potassium. Après 15 minutes d'agitation à 0°C .est ajouté 0,12 g de chlorure de 4-isopropoxybenzènesulfonyle. Après 6 heures d'agitation à 20°C, le mélange est hydrolysé par une solution saturée de $NH_4Cl$ puis extrait à l'AcOEt. La phase organique est lavée à l'eau, séchée sur du $MgSO_4$, filtrée puis évaporée à sec, purifiée par chromatographie sur gel de silice en éluant avec un mélange dichlorométhane/méthanol. Le produit est ensuite agité dans une solution d'éther chlorhydrique 2N et est ensuite filtré pour obtenir le composé désiré sous forme d'une poudre blanche.
F = 210 °C
$MH^+$ = 597 ; tr = 6,26 mn (Méthode M1)

<u>EXEMPLE 6 : Composé N°21</u>

3-(2-Fluorophényl)-5-méthoxy-1-[(4-méthoxy-3-méthylphényl)sulfonyl]-3-[4-(4-méthylpipérazin-1-yl)butyl]-1,3-dihydro-2*H*-pyrrolo[3,2-*b*]pyridin-2-one, isomère unique.

A une solution de 185 mg du Composé N°17 dans 1 ml de MeOH et 2 ml de DCM, sont ajoutés à 0° C 60 µl d'une solution aqueuse de formaldéhyde à 37 %, 20 µl d'AcOH, puis après 10 min 179 mg de $NaBH(OAC)_3$. Le mélange est agité pendant 18 heures avec retour à TA, dilué .avec 30 ml d'AcOEt et 20 ml d'une solution aqueuse de $NaHCO_3$ à 10 %. La phase organique est lavée par une solution aqueuse saturée de NaCl, séchée sur $MgSO_4$, filtrée puis évaporée à sec. Le résidu est purifié par chromatographie sur gel de silice en éluant avec un mélange dichlorométhane/méthanol additionné de 0,2 % d'ammoniaque à 20 %, pour conduire au composé attendu sous forme d'une résine blanche.
$MH^+$ = 597 ; tr = 5,91 mn (M4)

<u>EXEMPLE 7 : Composé N°15</u>

Chlorhydrate de 3-(2-fluorophényl)-1-[(4-isopropoxyphényl)sulfonyl]-5-méthyl-3-(3-pipérazin-1-ylpropyl)-1,3-dihydro-2*H*-pyrrolo[3,2-*b*]pyridin-2-one, isomère unique.

En utilisant la méthode décrite dans l'exemple 1, à partir de l'isomère dextrogyre obtenu à la préparation 4.6, on obtient le composé désiré sous forme d'une poudre blanche.
F = 150°C
MH+ = 567; tr = 5,73 mn (M4)

Les tableaux qui suivent illustrent les structures chimiques et les propriétés physiques de quelques exemples de composés selon l'invention, obtenus en suivant les modes opératoires décrits dans les Exemples ci-dessus.

Dans ces tableaux :

- Me, Et et iPr représentent respectivement des groupes méthyle, éthyle et isopropyle.

TABLEAU I

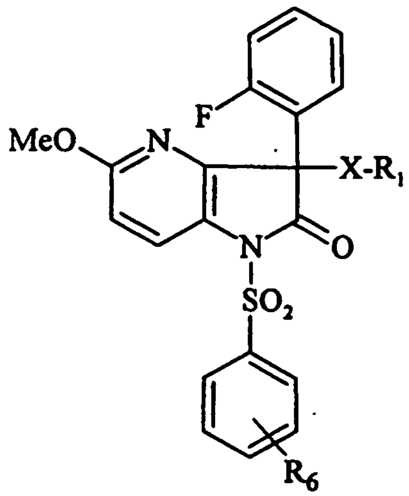

(I) $R_2$ = OMe
$R_3$ = H
$R_4$ = F
$R_5$ = H

$R_7$ = H

| Composés N° | X | $R_1$ | $R_6$ | Sels / Base 20 $\alpha_D^{20}$ (c; solvant) | F°C $MH^+$ ; tr (conditions) |
|---|---|---|---|---|---|
| 1 | $-(H_2)_3-$ | 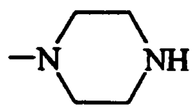 | 4-OiPr | HCl - 32,8 (5 mg/ml ; EtOAc) | 131°C 583,2 ; 6,42 (M1) |
| 2 | $-(CH_2)_3-$ | | 4-OiPr | HCl + 30,4 (5,2 mg/ml ; EtOAc) | 123°C 583,2 ; 6,42 (M1) |
| 3 | $-(H_2)_3-$ | 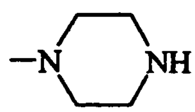 | 3-OMe | HCl + 21,4 (5,7 mg/ml ; EtOAc) | 178°C 555,4 ; 5,49 (M1) |
| 4 | $-(CH_2)_3-$ | 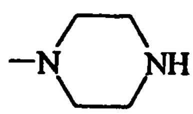 | 3-Cl | HCl + 34,1 (5,7 mg/ml ; EtOAc) | 177°C 559,2 ; 6,24 (M1) |
| 5 | $-(CH_2)_3-$ | | 4-OiPr | HCl - 36,4 (5,0 mg/ml ; EtOAc) | 119°C ; 582,4 ; 6,57 (M1) |
| 6 | $-(CH_2)_3-$ | 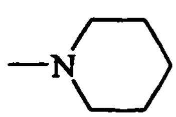 | 4-OiPr | HCl +38,1 (5,2 mg/ml ; EtOAc) | 157°C 582,4 ; 6,57 (M1) |

(suite)

| Composés N° | X | $R_1$ | $R_6$ | Sels / Base 20 $\alpha_D^{20}$ (c; solvant) | F°C $MH^+$ ; tr (conditions) |
|---|---|---|---|---|---|
| 7 | $-(CH_2)_3-$ | 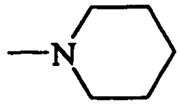 | 3-OMe | HCl + 45,2 (5,9 mg/ml; EtOAc) | 148°C 554,1 ; 6,59 (M1) |
| 8 | $-(CH_2)_3-$ | 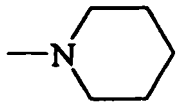 | $4-OCHF_2$ | HCl + 44,2 (5,2 mg/ ml ; EtOAc) | 133°C 590,4 ; 6,27 (M1) |
| 9 | $-(CH_2)_3-$ | 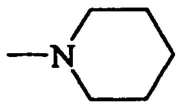 | 3-Cl | HCl + 44,3 (5,1 mg/ ml ; EtOAc) | 148°C 558,2 ; 6,81 (M1) |
| 10 | -(CH2) 4- | 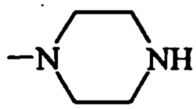 | 4-OiPr | HCl (+/-) | 210°C 597,0 ; 6,26 (M1) |
| 11 | $-(CH_2)_3-$ | 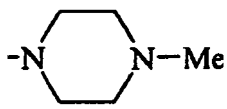 | 4-OiPr | HCl +14 (0,5 ; MeOH) | 190°C 597 ; 6,24 (M4) |
| 12 | $-(CH_2)_4-$ | 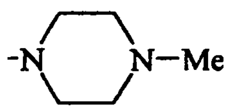 | 4-OiPr | +49,2 (0,51 ; AcOEt) | 52°C 611 ; 6,14 (M4) |
| 13 | $-(CH_2)_3-$ | 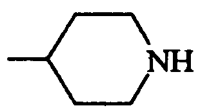 | 4-OiPr | HCl +14,5 (0,5 ; MeOH) | 582 ; 7,01 (M4) |
| 14 | $-(CH_2)_4-$ | 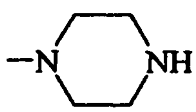 | 4-OiPr | HCl +10,5 (0,5 ; MeOH) | 133°C 597 ; 5,78 (M4) |

**TABLEAU II**

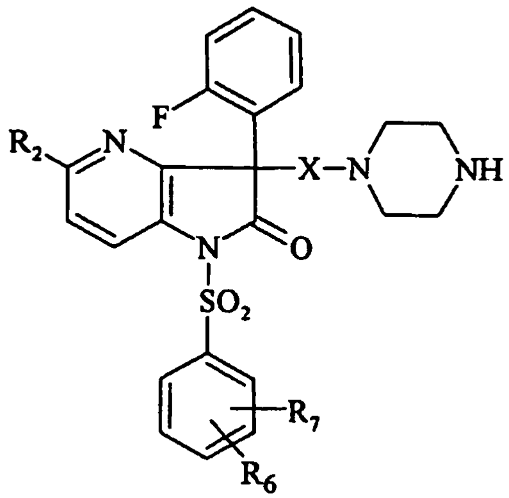

(I) $R_1$ = 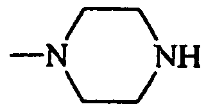

$R_3$ = H

$R_4$ = F

$R_5$ = H

| Composés N° | X | $R_2$ | $R_6$ | $R_7$ | Sels / Base (c ; solvant) (c ; | F°C $\alpha_D^{20}$ $MH^+$ ; tr (conditions) |
|---|---|---|---|---|---|---|
| 15 | $-(CH_2)_3-$ | Me | 4-OiPr | H | HCl +16,8 (0,5 ; MeOH) | 150°C 567 ; 5,73 (M4) |
| 17 | $-(CH_2)_4-$ | OMe | 4-OMe | 3-Me | HCl + 35,7 (0,51 ; MeOH) | 158°C 583 ; 5,58 (M4) |
| 18 | $-(CH_2)_3-$ | Me | 4-OMe | 2-OMe | HCl + 47 (0,5 ; MeOH) | 176°C 569 ; 5,16 (M4) |
| 19 | $-(CH_2)_3-$ | OMe | 4-OMe | 3-Me | HCl + 47,2 (0,5 ; MeOH) | 160°C 569 ; 6,13 (M4) |
| 20 | $-(CH_2)_3-$ | OMe | 4-OMe | 2-OMe | HCl + 35,4 (0,5 ; MeOH) | 170°C 858 ; 5,85 (M4) |
| 22 | $-(CH_2)_3-$ | Me | 4-OMe | 3-Me | HCl è + 56,7 (0,51 ; MeOH) | 162°C 553 ; 5,51 (M4) |
| 23 | $-(CH_2)_4-$ | OMe | 4-OMe | 2-OMe | HCl . + 35,8 (0,52 ; MeOH) | 170°C 599 ; 5,25 (M4) |

**TABLEAU III**

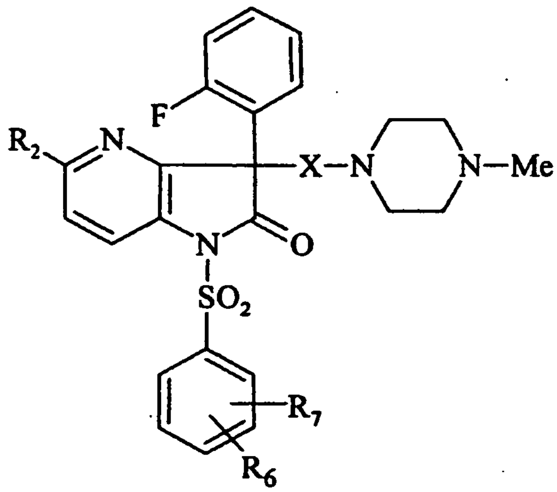

(I) $R_1$ = 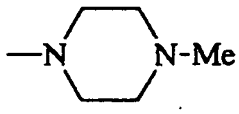

$R_3$ = H

$R_4$ = F

$R_5$ = H

| Composés N° | X | $R_2$ | $R_6$ | $R_7$ | Sels / Base $\alpha_D^{20}$ (c ; solvant) | F°C MH$^+$ ; tr (conditions) |
|---|---|---|---|---|---|---|
| 16 | $-(CH_2)_3-$ | Me | 4-OiPr | H | HCl +16,9 (0,5 ; MeOH) | 218°C 581 ; 5,96 (M4) |
| 21 | $-(CH_2)_4-$ | OMe | 4-OMe | 3-Me | - + 36,5 (0,49 ; AcOEt) | 55°C 597 ; 5,91 (M4) |

Les composés selon l'invention ont fait l'objet d'essais pharmacologiques.

L'affinité des composés de formule (I) selon l'invention pour les récepteurs $V_{1a}$ de l'arginine-vasopressine a été déterminée in vitro en utilisant la méthode décrite par M. Thibonnier et al., J. Biol. Chem., 1994, 269, 3304-3310, Cette méthode consiste à étudier in vitro le déplacement de l'arginine-vasopressine tritiée ([3H]-AVP) aux récepteurs $V_{1a}$ présents sur des préparations membranaires ou cellulaires portant les récepteurs $V_{1a}$ de rat ou humains. Les composés de formule (I) présentent une affinité pour les récepteurs $V_{1a}$ humains de l'arginine-vasopressine avec des ($CI_{50}$) (concentration inhibitrice de 50 % de la liaison de l'arginine-vasopressine tritiée ([3H]-AVP à ses récepteurs) généralement inférieures à 10 nanomolaire (10-8 M) dans ce dernier test.

L'affinité des composés de formule (I) selon l'invention pour les récepteurs $V_{1b}$ de l'arginine-vasopressine a été déterminée in vitro en utilisant la méthode décrite par Y. de Keyser et al. dans Febs Letters, 1994, 356, 215-220, Cette méthode consiste à étudier in vitro le déplacement de l'arginine-vasopressine tritiée ([3H]-AVP) aux récepteurs $V_{1b}$ présents sur des préparations cellulaires portant les récepteurs $V_{1b}$ humains. Certains composés étudiés présentent en plus une affinité pour les récepteurs $V_{1b}$ humains avec des ($CI_{50}$) inférieures à 30 nanomolaire (3.10-8 M) dans ce dernier test.

L'affinité des composés de formule (I) selon l'invention pour les récepteurs $V_2$ de la vasopressine a également été étudiée (méthode décrite par M. Birnbaumer et al. dans Nature (Lond.), 1992, 357, 333-335). Les composés étudiés sont peu ou pas affins pour les récepteurs $V_2$ humains avec des $CI_{50}$ supérieures à $10^{-7}$ M.

L'affinité des composés selon l'invention pour les récepteurs de l'ocytocine a été déterminée dans un test de liaison *in vitro* en utilisant la méthode décrite par J. Elands et al. dans Eur. J. Pharmacol., 1987, 147, 197-207. Cette méthode consiste à étudier *in vitro* le déplacement d'un analogue radioiodé de l'ocytocine aux récepteurs de l'ocytocine dans une préparation membranaire de cellules transfectée avec le récepteur ocytocique humain utérin.

Les composés étudiés sont peu ou pas affins pour les récepteurs humains de l'ocytocine avec des $CI_{50}$ généralement supérieures à $10^{-7}$ M.

Le TABLEAU IV qui suit illustre les résultats pharmacologiques comparatifs des composés N° 2, N° 4 et N° 11 selon l'invention avec les composés $\alpha$ et $\beta$ de l'art antérieur sur les différents tests *in-vitro* mesurant l'affinité aux récepteurs $V_{1a}$, $V_{1b}$, $V_2$ et Ocytocine humains. Les résultats sont exprimés par la concentration inhibitrice de 50% ($CI_{50}$) en nanomolaire (nM).

**TABLEAU IV**

| Composé N° : | $CI_{50}$ $V_{1a}$ (nM) | $CI_{50}$ $V_{1b}$ (nM) | $CI_{50}$ $V_2$ (nM) | $CI_{50}$ OT (nM) |
|---|---|---|---|---|
| 2 | 0,82 | 350 | 120 | 71 |
| 4 | 8,9 | >1000 | >1000 | >1000 |
| 11 | 7,1 | >1000 | 210 | 100 |
| Composé α | 23 | 210 | 19 | 100 |
| Composé β | 18 | 180 | 13 | 69 |

Le caractère agoniste ou antagoniste des composés est déterminé *in vitro* dans un test de mesure de calcium intracellulaire (FLIPR) sur cellules exprimant les récepteurs V1a humains selon la technique générale décrite dans Sullivan et al, Methods Mol. Biol., 1999, 114, 125-133, en utilisant 1 μM de Fluo-4 AM et dans un test d'agrégation plaquettaire induite à l'AVP sur PRP (plasma riche en plaquettes) humain selon la méthodologie décrite dans J. Clin. Invest., 1993, 92, 224.231. Les composés sont préincubés 30 minutes avant l'addition de l'arginine vasopressine afin de déterminer les propriétés agonistes et antagonistes de ces molécules. Les $CI_{50}$ des composés selon l'invention pour les récepteurs $V_{1a}$ mesurées dans ces études sont faibles (inférieures à $2.10^{-8}$ M).

Ces résultats pharmacologiques montrent que les composés selon l'invention, en particulier les composés N° 2 et N° 4 sont des antagonistes des récepteurs $V_{1a}$ en bloquant les effets pharmacologiques provoqués par l'arginine-vasopressine.

Les composés selon l'invention peuvent donc être utilisés pour la préparation de médicaments, en particulier de médicaments antagonistes des récepteurs $V_{1a}$ humains de l'arginine-vasopressine et en plus pour certains composés des récepteurs $V_{1b}$ humains de l'AVP.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, et sont avantageusement utiles dans les désordres de la sphère urogénitale notamment dans les domaines obstétrique et gynécologique, notamment comme agent tocolytique ou relaxant utérin ou pour contrôler l'hyperactivité utérine, les contractions de l'utérus avant que la grossesse soit arrivée à terme, pour contrôler le travail prénatal, ou encore contrôler le travail préparatoire en vue d'un accouchement par césarienne, favoriser la croissance du foetus in-utero, diminuer le stress et l'anoxie au moment des contractions, pour résoudre les problèmes de stérilité, fertilité, contrôler les naissances (usage vétérinaire en particulier), contrôler l'oestrus, le sevrage, le transfert et l'implantation d'embryon lors de la fécondation in vitro ; traiter l'endométriose, les dysménorrhées, ainsi que l'incontinence urinaire à l'effort ou d'urgence, l'hypertrophie bénigne de la prostate, les désordres de la miction, les infections urogénitales, les lithiases urinaires et les dysfonctions érectiles

Ces médicaments sont également utiles dans le traitement ou la prévention de différentes affections vasopressine-dépendantes tels que les affections cardiovasculaires, comme l'hypertension, l'hypertension pulmonaire, l'insuffisance cardiaque, l'infarctus du myocarde, ou le vasospasme coronaire, en particulier chez le fumeur, la maladie de Raynaud, les angines instables et PTCA (de l'anglais percutaneous transluminal coronary angioplasty), l'ischémie cardiaque, les dérèglements de l'hémostase, la thrombose ; les affections du système nerveux central comme la migraine, le vasospasme cérébral, l'hémorragie cérébrale, les traumatismes et les oedèmes cérébraux, la dépression, l'anxiété, le stress, les troubles émotionnels, le trouble obsessionnel-compulsif, la schizophrénie, les attaques de panique, les états psychotiques, l'agressivité, les troubles de la mémoire, du sommeil, les désordres cognitifs par exemple ; les affections du système rénal comme le vasospasme rénal, la nécrose du cortex rénal ; le diabète insipide néphrogénique ; la néphropathie diabétique ; les polykystoses rénales, les affections du système gastrique comme le vasospasme gastrique, l'hépatocirrhose, les ulcères, la pathologie des vomissements, par exemple la nausée y compris la nausée due à une chimiothérapie, le mal des transports. Les composés selon l'invention peuvent également être utilisés dans le traitement des troubles du comportement sexuel ; chez la femme, les composés selon l'invention peuvent être utilisés pour traiter la dysménorrhée primaire et secondaire, le travail prématuré ou l'endométriose. On peut également utiliser les composés selon l'invention dans le traitement des cancers comme les cancers pulmonaires à petites cellules ou les cancers mammaires ; des encéphalopathies hyponatrémiques ; du syndrome pulmonaire, de la maladie de Ménière ; des hypertensions oculaires, du glaucome, de la cataracte ; de l'obésité ; du diabète de type I et II ; de l'athérosclérose ; du syndrome métabolique ; de l'hyperlipidémie ; de la résistance à l'insuline ; de l'hypertriglycéridémie ; dans les traitements post-opératoires, notamment après une chirurgie abdominale ; de l'autisme ; de l'hypercortisolémie ; de l'hyperaldostéronémie ; des phéochromocytomes ; du syndrome de Cushing ; de la preeclempsie ; des désordres de la micturation ; de l'éjaculation prématurée.

Les composés selon l'invention peuvent aussi être utilisés dans le traitement ou la prévention de toutes les pathologies consécutives au stress comme la fatigue et ses syndromes, les désordres ACTH dépendants, les troubles cardiaques, la douleur, les modifications de la vidange gastrique, de l'excrétion fécale (colite, syndrome du colon irritable, maladie de Crohn), de la sécrétion acide, l'hyperglycémie, l'immunosuppression, les processus inflammatoires (arthrite rhumatoïde et ostéoarthrite), les infections multiples, le choc septique, les cancers, l'asthme, le psoriasis, les allergies et les désordres neuropsychiatriques variés tel que l'anorexie nerveuse, la boulimie, les troubles de l'humeur, la dépression, l'anxiété, les troubles du sommeil, les états de panique, les phobies, l'obsession, les troubles de la perception de la douleur (fibromyalgie), les maladies neurodégénératrices (maladie d'Alzheimer, maladie de Parkinson, maladie d'Huntington), la dépendance à une substance (alcool ou drogue), le sevrage, le stress hémorragique, les spasmes musculaires, l'hypoglycémie. Les composés selon l'invention peuvent également être utilisés dans le traitement ou la prévention des états de stress chronique comme l'immunodépression, les troubles de la fertilité, les dysfonctionnements de l'axe hypothalamo-hypophysosurrénalien.

Les composés selon l'invention peuvent également être utilisés comme psychostimulants, provoquant l'augmentation de l'éveil, la réactivité émotionnelle face à l'environnement et facilitant l'adaptation.

Les composés selon la présente invention peuvent enfin être utilisés dans la cicatrisation, dans l'analgésie, dans l'anxiolyse, dans la prévention de la douleur, dans la prévention de l'anxiété, la dépression, la schizophrénie, l'autisme, les syndromes obsessionnels compulsifs, dans le comportement maternel (facilitation de la reconnaissance et de l'acceptation de la mère par l'enfant) et social, la mémoire ; la régulation de la prise de nourriture et de boisson, la dépendance aux drogues, le sevrage et la motivation sexuelle ; l'hypertension, l'hyponatrémie, l'insuffisance cardiaque, l'arthérosclérose, l'angiogénèse, la prolifération de tumeurs, le sarcome de Kaposi, réguler le stockage des graisses par l'adipocyte, contrôler les hyperlipidémies, triglycéridémie et le syndrome métabolique.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

**1.** Composé répondant à la formule (I):

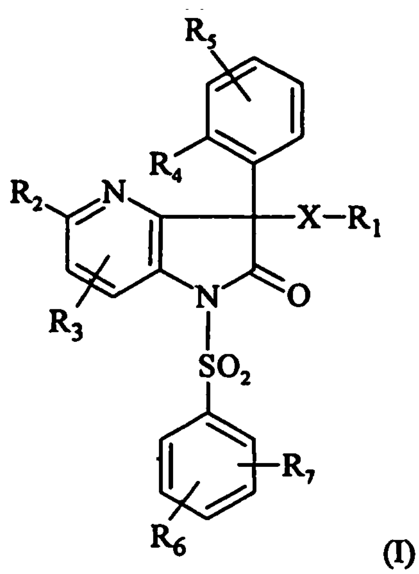

(I)

dans laquelle :

- X représente un radical bivalent $(C_1-C_5)$alkylène non substitué ou substitué une ou plusieurs fois sur un atome de carbone par un atome de fluor ou par un $(C_1-C_3)$alkyle ;
- $R_1$ représente :

  . un groupe $-NR_8R_9$ ;
  . un radical pipéridin-3-yle ou pipéridin-4-yle non substitué ou substitué une ou plusieurs fois par un $(C_1-C_4)$ alkyle, un $(C_3-C_5)$cycloalkyle, les atomes de carbone pouvant être également substitués par un ou plusieurs atomes de fluor ;

- $R_2$ représente un atome d'hydrogène, un atome d'halogène, un groupe Alk, un groupe OAlk ;
- $R_3$ représente un atome d'hydrogène, un atome d'halogène, un groupe Alk, un groupe OAlk ;
- $R_4$ représente un atome d'hydrogène, un atome d'halogène, un groupe Alk, un hydroxy, un groupe OAlk ;
- $R_5$ représente un atome d'hydrogène, un atome d'halogène, un groupe Alk, un hydroxy, un groupe OAlk ;
- $R_6$ représente un atome d'hydrogène, un atome d'halogène, un groupe Alk, un hydroxy, un groupe OAlk ;
- $R_7$ représente un atome d'hydrogène, un atome d'halogène, un groupe Alk, un hydroxy, un groupe OAlk ;
- $R_8$ et $R_9$ représentent chacun indépendamment un atome d'hydrogène ou un $(C_1-C_4)$alkyle ;
- ou bien $R_8$ et $R_9$ ensemble avec l'atome d'azote auquel ils sont liés constituent un radical hétérocyclique choisi parmi : l'azétidin-1-yle, la pyrrolidin-1-yle, la pipéridin-1-yle, la morpholin-4-yle, la thiomorpholin-4-yle, la pipérazin-1-yle ou la perhydroazépin-1-yle, ledit radical hétérocyclique étant non substitué ou substitué une ou plusieurs fois par un amino, un hydroxy, un $(C_1-C_4)$ alkyle, un $(C_3-C_5)$cycloalkyle, un $(C_1-C_4)$alcoxy, les atomes de carbone pouvant être également substitués par un ou plusieurs atomes de fluor ;
- Alk représente un $(C_1-C_4)$alkyle non substitué ou substitué une ou plusieurs fois par un atome de fluor.

à l'état de base ou de sel d'addition à un acide.

**2.** Composés de formule (I) selon la revendication 1 dans laquelle :

- X représente un radical bivalent $(C_3-C_4)$ alkylène non substitué ou substitué une ou plusieurs fois sur un atome de carbone par un atome de fluor ou par un $(C_1-C_3)$ alkyle ;
- $R_1$ représente :

  . un groupe $-NR_8R_9$ ;

. un radical pipéridin-4-yle ;

- $R_2$ représente un groupe Alk, un groupe OAlk ;
- $R_3$ représente un atome d'hydrogène ;
- $R_4$ représente un atome d'halogène ;
- $R_5$ représente un atome d'hydrogène ;
- $R_6$ représente un atome d'halogène, un groupe OAlk ;
- $R_7$ représente un atome d'hydrogène, un groupe Alk, un groupe OAlk ;
- $R_8$ et $R_9$ ensemble avec l'atome d'azote auquel ils sont liés constituent un radical hétérocyclique choisi parmi : la pipéridin-1-yle, la pipérazin-1-yle, ledit radical hétérocyclique étant non substitué ou substitué une ou deux fois par un atome de fluor, un amino, un hydroxy, un méthyle ;
- Alk représente un ($C_1$-$C_4$)alkyle non substitué ou substitué une ou plusieurs fois par un atome de fluor ;

à l'état de base ou de sel d'addition à un acide.

**3.** Composés de formule (I) selon la revendication 1 dans laquelle :

- X représente un radical bivalent triméthylène ou tétraméthylène ;
- $R_1$ représente :

. un pipéridin-1-yle, un pipérazin-1-yle, un 4-méthylpipérazin-1-yle ;
. un pipéridin-4-yle ;

- $R_2$ représente un méthyle, un méthoxy ;
- $R_3$ représente un atome d'hydrogène ;
- $R_4$ représente un atome de fluor ;
- $R_5$ représente un atome d'hydrogène ;
- $R_6$ représente un atome de chlore, un méthoxy, un isopropoxy, un difluorométhoxy ;
- $R_7$ représente un atome d'hydrogène, un méthyle, un méthoxy ;

à l'état de base ou de sel d'addition à un acide.

**4.** Composé selon la revendication 1 de formule (I) choisi parmi :

- 3-(2-Fluorophényl)-1-[(4-isopropoxyphényl)sulfonyl]-5-méthoxy-3-(3-pipérazin-1-ylpropyl)-1,3-dihydro-2*H*-pyrrolo[3,2-*b*]pyridin-2-one;
- 3-(2-Fluorophényl)-1-[(4-isopropoxyphényl)sulfonyl]-5-méthoxy-3-(3-pipérazin-1-ylpropyl)-1,3-dihydro-2*H*-pyrrolo[3,2-*b*]pyridin-2-one ;
- 3-(2-Fluorophényl)-5-méthoxy-1-[(3-méthoxyphényl)sulfonyl]-3-(3-pipérazin-1-ylpropyl)-1,3-dihydro-2*H*-pyrrolo[3,2-*b*]pyridin-2-one ;
- 1-[(3-Chlorophényl)sulfonyl]-3-(2-fluorophényl)-5-méthoxy-3-(3-pipérazin-1-ylpropyl)-1,3-dihydro-2*H*-pyrrolo[3,2-*b*]pyridin-2-one ;
- 3-(2-Fluorophényl)-1-[(4-isopropoxyphényl)sulfonyl]-5-méthoxy-3-(3-pipéridin-1-ylpropyl)-1,3-dihydro-2*H*-pyrrolo[3,2-*b*]pyridin-2-one ;
- 3-(2-Fluorophényl)-1-[(4-isopropoxyphényl)sulfonyl]-5-méthoxy-3-(3-pipéridin-1-ylpropyl)-1,3-dihydro-2*H*-pyrrolo[3,2-*b*]pyridin-2-one ;
- 3-(2-Fluorophényl)-5-méthoxy-1-[(3-méthoxyphényl)sulfonyl]-3-(3-pipéridin-1-ylpropyl)-1,3-dihydro-2*H*-pyrrolo[3,2-*b*]pyridin-2-one, isomère ;
- 1-{[4-(Difluorométhoxy)phényl]sulfonyl}-3-(2-fluorophényl)-5-méthoxy-3-(3-pipéridin-1-ylpropyl)-1,3-dihydro-2*H*-pyrrolo[3,2-*b*]pyridin-2-one ;
- 1-[(3-Chlorophényl)sulfonyl]-3-(2-fluorophényl)-5-méthoxy-3-(3-pipéridin-1-ylpropyl)-1,3-dihydro-2*H*-pyrrolo[3,2-*b*]pyridin-2-one ;
- 3-(2-Fluorophényl)-1-[(4-isopropoxyphényl)sulfonyl]-5-méthoxy-3-(4-pipérazin-1-ylbutyl)-1,3-dihydro-2*H*-pyrrolo[3,2-*b*]pyridin-2-one;
- 3-(2-Fluorophényl)-[(4-isopropoxyphényl)sulfonyl]-5-méthoxy-3-[3-(4-méthylpipérazin-1-yl)propyl]-1,3-dihydro-2*H*-pyrrolo[3,2-*b*]pyridin-2-one ;
- 3-(2-Fluorophényl)-1-[(4-isopropoxyphényl)sulfonyl]-5-méthoxy-3-[4-(4-méthylpipérazin-1-yl)butyl]-1,3-dihydro-2*H*-pyrrolo[3,2-*b*]pyridin-2-one ;
- 3-(2-Fluorophényl)-1-[(4-isopropoxyphényl)sulfonyl]-5-méthoxy-3-(3-pipéridin-4-yl propyl)-1,3-dihydro-2*H*-pyrrolo[3,2-*b*]pyridin-2-one ;

-3-(2-Fluorophényl)-1-[(4-isopropoxyphényl)sulfonyl]-5-méthoxy-3-(4-pipérazin-1-yl butyl)-1,3-dihydro-2*H*-pyrrolo[3,2-*b*]pyridin-2-one ;
- 3-(2-Fluorophényl)-1-[(4-isopropoxyphényl)sulfonyl]-5-méthyl-3-(3-pipérazin-1-yl propyl)-1,3-dihydro-2*H*-pyrrolo[3,2-*b*]pyridin-2-one ;
- 3-(2-Fluorophényl)-5-méthoxy-1-[(4-méthoxy-3-méthylphényl)sulfonyl]-3-(4-pipérazin-1-yl butyl)-1,3-dihydro-2*H*-pyrrolo[3,2-*b*]pyridin-2-one ;
-1-[(2,4-Diméthoxyphényl)sulfonyl]-3-(2-fluorophényl)-5-méthyl-3-(3-pipérazin-1-ylpropyl)-1,3-dihydro-2*H*-pyrrolo[3,2-*b*]pyridin-2-one ;
- 3-(2-Fluorophényl)-5-méthoxy-1-[(4-méthoxy-3-méthylphényl)sulfonyl]-3-(3-pipérazin-1-yl propyl)-1,3-dihydro-2*H*-pyrrolo[3,2-*b*]pyridin-2-one ;
- 1-[(2,4-Diméthoxyphényl)sulfonyl]-3-(2-fluorophényl)-5-méthoxy-3-(3-pipérazin-1-yl propyl)-1,3-dihydro-2*H*-pyrrolo[3,2-*b*]pyridin-2-one ;
- 3-(2-Fluorophényl)-1-[(4-méthoxy-3-méthylphényl)sulfonyl]-5-méthyl-3-(3-pipérazin-1-yl propyl)-1,3-dihydro-2*H*-pyrrolo[3,2-*b*]pyridin-2-one ;
- 1-[(2,4-Diméthoxyphényl)sulfonyl]-3-(2-fluorophényl)-5-méthoxy-3-(4-pipérazin-1-yl butyl)-1,3-dihydro-2*H*-pyrrolo[3,2-*b*]pyridin-2-one ;
- 3-(2-Fluorophényl)-1-[(4-isopropoxyphényl)sulfonyl]-5-méthyl-3-[3-(4-méthylpipérazin-1-yl)propyl]-1,3-dihydro-2*H*-pyrrolo[3,2-*b*]pyridin-2-one ;
- 3-(2-Fluorophényl)-5-méthoxy-1-[(4-méthoxy-3-méthylphényl)sulfonyl]-3-[4-(4-méthylpipérazin-1-yl)butyl)-1,3-dihydro-2*H*-pyrrolo[3,2-*b*]pyridin-2-one ; à l'état de base ou de sels d'addition à des acides ; sous forme d'énantiomères purs ou de mélange racémique.

**5.** Procédé de préparation des composés de formule (I) selon l'une quelconque des revendications 1 à 4, **caractérisé en ce que** :

On fait réagir, en présence d'une base, un composé de formule :

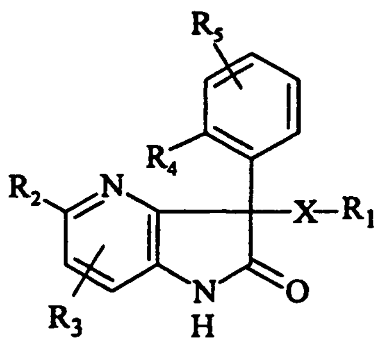

(II)

dans laquelle X, $R_1$, $R_2$, $R_3$, $R_4$ et $R_5$ sont tels que définis pour un composé de formule (I), avec un halogénure de sulfonyle de formule :

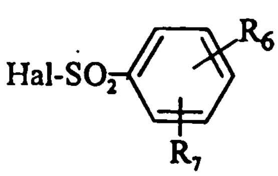

(III)

dans laquelle $R_6$ et $R_7$ sont tels que définis pour un composé de formule (I) et Hal représente un atome d'halogène.

**6.** Médicament **caractérisé en ce qu'**il comprend un composé de formule (I) selon l'une quelconque des revendications 1 à 4, ou un sel d'addition de ce composé à un acide pharmaceutiquement acceptable.

**7.** Composition pharmaceutique **caractérisée en ce qu'**elle comprend un composé de formule (I) selon l'une quelconque des revendications 1 à 4, ou un sel pharmaceutiquement acceptable, ainsi qu'au moins un excipient pharmaceutiquement acceptable.

## Claims

**1.** Compound corresponding to the formula (I):

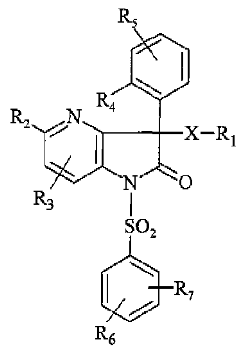

(I)

in which:

- X represents a bivalent $(C_1-C_5)$alkylene radical that is unsubstituted or substituted once or several times on a carbon atom with a fluorine atom or with a $(C_1-C_3)$alkyl;
- $R_1$ represents:

  . a group $-NR_8R_9$;
  . a piperidin-3-yl or piperidin-4-yl radical that is unsubstituted or substituted once or several times with a $(C_1-C_4)$alkyl, a $(C_3-C_5)$cycloalkyl, it being possible for the carbon atoms to also be substituted with one or more fluorine atoms;

- $R_2$ represents a hydrogen atom, a halogen atom, an Alk group, an OAlk group;
- $R_3$ represents a hydrogen atom, a halogen atom, an Alk group, an OAlk group;
- $R_4$ represents a hydrogen atom, a halogen atom, an Alk group, a hydroxyl, an OAlk group;
- $R_5$ represents a hydrogen atom, a halogen atom, an Alk group, a hydroxyl, an OAlk group;
- $R_6$ represents a hydrogen atom, a halogen atom, an Alk group, a hydroxyl, an OAlk group;
- $R_7$ represents a hydrogen atom, a halogen atom, an Alk group, a hydroxyl, an OAlk group;
- $R_8$ and $R_9$ each independently represent a hydrogen atom or a $(C_1-C_4)$alkyl;
- or alternatively $R_8$ and $R_9$, together with the nitrogen atom to which they are attached, constitute a heterocyclic radical chosen from: azetidin-1-yl, pyrrolidin-1-yl, piperidin-1-yl, morpholin-4-yl, thiomorpholin-4-yl, piperazin-1-yl or perhydroazepin-1-yl, the said heterocyclic radical being unsubstituted or substituted once or several times with an amino, a hydroxyl, a $(C_1-C_4)$alkyl, a $(C_3-C_5)$cycloalkyl, a $(C_1-C_4)$alkoxy, it being possible for the carbon atoms to also be substituted with one or more fluorine atoms;
- Alk represents a $(C_1-C_4)$alkyl that is unsubstituted or substituted once or several times with a fluorine atom, in the form of a base or of an addition salt with an acid.

**2.** Compounds of formula (I) according to Claim 1, in which:

- X represents a bivalent $(C_3-C_4)$ alkylene radical that is unsubstituted or substituted once or several times on a carbon atom with a fluorine atom or with a $(C_1-C_3)$ alkyl;
- $R_1$ represents:

  . a group $-NR_8R_9$;
  . a piperidin-4-yl radical;

- $R_2$ represents an Alk group, an OAlk group;
- $R_3$ represents a hydrogen atom;
- $R_4$ represents a halogen atom;

- $R_5$ represents a hydrogen atom;
- $R_6$ represents a halogen atom, an OAlk group;
- $R_7$ represents a hydrogen atom, an Alk group, an OAlk group;
- $R_8$ and $R_9$ together with the nitrogen atom to which they are attached constitute a heterocyclic radical chosen from: piperidin-1-yl, piperazin-1-yl, the said heterocyclic radical being unsubstituted or substituted once or twice with a fluorine atom, an amino, a hydroxyl, a methyl;
- Alk represents a ($C_1$-$C_4$)alkyl that is unsubstituted or substituted once or several times with a fluorine atom;

in the form of a base or an addition salt with an acid.

**3.** Compounds of formula (I) according to Claim 1, in which:

- X represents a bivalent trimethylene or tetramethylene radical;
- $R_1$ represents:

  . a piperidin-1-yl, a piperazin-1-yl, a 4-methylpiperazin-1-yl;
  . a piperidin-4-yl;

- $R_2$ represents a methyl, a methoxy;
- $R_3$ represents a hydrogen atom;
- $R_4$ represents a fluorine atom;
- $R_5$ represents a hydrogen atom;
- $R_6$ represents a chlorine atom, a methoxy, an isopropoxy, a difluoromethoxy;
- $R_7$ represents a hydrogen atom, a methyl, a methoxy;

in the form of a base or an addition salt with an acid.

**4.** Compound according to Claim 1 of formula (I), chosen from:

- 3-(2-fluorophenyl)-1-[(4-isopropoxyphenyl)sulphonyl]-5-methoxy-3-(3-piperazin-1-ylpropyl)-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-(3-piperazin-1-ylpropyl)-1,3-dihydro-2*H*-pyrrolo[3,2-*b*]pyridin-2-one;
- 3-(2-fluorophenyl)-5-methoxy-1-[(3-methoxyphenyl)sulphonyl]-3-(3-piperazin-1-ylpropyl)-1,3-dihydro-2*H*-pyrrolo[3,2-*b*]pyridin-2-one;
- 1-[(3-chlorophenyl)sulphonyl]-3-(2-fluorophenyl)-5-methoxy-3-(3-piperazin-1-ylpropyl)-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-(3-piperidin-1-ylpropyl)-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-(3-piperidin-1-ylpropyl)-1,3-dihydro-2*H*-pyrrolo[3,2-*b*]pyridin-2-one;
- 3-(2-fluorophenyl)-5-methoxy-1-[(3-methoxyphenyl)sulphonyl]-3-(3-piperidin-1-ylpropyl)-1,3-dihydro-2*H*-pyrrolo[3,2-*b*]pyridin-2-one, isomer;
- 1-{[4-(difluoromethoxy)phenyl]sulphonyl}-3-(2-fluorophenyl)-5-methoxy-3-(3-piperidin-1-ylpropyl)-1,3-dihydro-2*H*-pyrrolo[3,2-*b*]pyridin-2-one;
- 1-[(3-chlorophenyl)sulphonyl]-3-(2-fluorophenyl)-5-methoxy-3-(3-piperidin-1-ylpropyl)-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-(4-piperazin-1-ylbutyl)-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-[3-(4-methylpiperazin-1-yl)propyl]-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-[4-(4-methylpiperazin-1-yl)butyl]-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-(3-piperidin-4-ylpropyl)-1,3-dihydro-2*H*-pyrrolo[3,2-*b*]pyridin-2-one;
- 3-(2-fluorophenyl)-1-[(4-isopropoxyphenyl)sulphonyl]-5-methoxy-3-(4-piperazin-1-ylbutyl)-1,3-dihydro-2*H*-pyrrolo[3,2-*b*]pyridin-2-one;
- 3-(2-fluorophenyl)-1-[(4-isopropoxyphenyl)sulphonyl]-5-methyl-3-(3-piperazin-1-ylpropyl)-1,3-dihydro-2*H*-pyrrolo[3,2-*b*]pyridin-2-one;
- 3-(2-fluorophenyl)-5-methoxy-1-[(4-methoxy-3-methylphenyl)sulphonyl]-3-(4-piperazin-1-ylbutyl)-1,3-dihydro-2*H*-pyrrolo[3,2-*b*]pyridin-2-one;
- 1-[(2,4-dimethoxyphenyl)sulphonyl]-3-(2-fluorophenyl)-5-methyl-3-(3-piperazin-1-ylpropyl)-1,3-dihydro-2*H*-pyrrolo[3,2-*b*]pyridin-2-one;
- 3-(2-fluorophenyl)-5-methoxy-1-[(4-methoxy-3-methylphenyl)sulphonyl]-3-(3-piperazin-1-ylpropyl)-1,3-dihydro-2*H*-pyrrolo[3,2-*b*]pyridin-2-one;
- 1-[(2,4-dimethoxyphenyl)sulphonyl]-3-(2-fluorophenyl)-5-methoxy-3-(3-piperazin-1-ylpropyl)-1,3-dihydro-2*H*-pyrrolo[3,2-*b*]pyridin-2-one;
- 3-(2-fluorophenyl)-1-[(4-methoxy-3-methylphenyl)sulphonyl]-5-methyl-3-(3-piperazin-1-ylpropyl)-1,3-dihydro-2*H*-pyrrolo[3,2-*b*]pyridin-2-one;
- 1-[(2,4-dimethoxyphenyl)sulphonyl]-3-(2-fluorophenyl)-5-methoxy-3-(4-piperazin-1-ylbutyl)-1,3-dihydro-2*H*-pyrrolo[3,2-*b*]pyridin-2-one;
- 3-(2-fluorophenyl)-1-[(4-isopropoxyphenyl)sulphonyl]-5-methyl-3-[3-(4-methylpiperazin-1-yl)propyl]-1,3-dihydro-2*H*-pyrrolo[3,2-*b*]pyridin-2-one;
- 3-(2-fluorophenyl)-5-methoxy-1-[(4-methoxy-3-methylphenyl)sulphonyl]-3-[4-(4-methylpiperazin-1-yl)butyl)-1,3-dihydro-2*H*-pyrrolo[3,2-*b*]pyridin-2-one;

in the form of a base or of addition salts with acids, in the form of pure enantiomers or of a racemic mixture.

**5.** Method for preparing the compounds of formula (I) according to any one of Claims 1 to 4, **characterized in that**:

a compound of the formula:

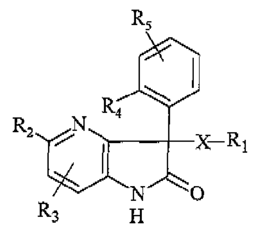

(II)

in which X, $R_1$, $R_2$, $R_3$, $R_4$ and $R_5$ are as defined for a compound of formula (I), is reacted, in the presence of a base, with a sulphonyl halide of formula:

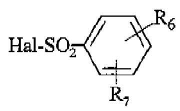

(III)

in which $R_6$ and $R_7$ are as defined for a compound of formula (I) and Hal represents a halogen atom.

**6.** Medicament, **characterized in that** it comprises a compound of formula (I) according to any one of Claims 1 to 4, or an addition salt of this compound with a pharmaceutically acceptable acid.

**7.** Pharmaceutical composition, **characterized in that** it comprises a compound of formula (I) according to any one of Claims 1 to 4, or a pharmaceutically acceptable salt, and at least one pharmaceutically acceptable excipient.

**Patentansprüche**

**1.** Verbindung der Formel (I):

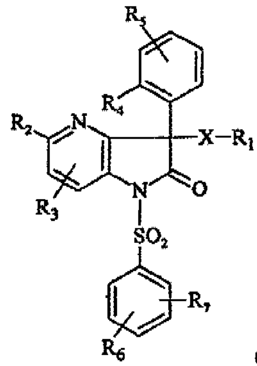

(I)

wobei:

- X für einen zweiwertigen ($C_1$-$C_5$)-Alkylenrest steht, der unsubstituiert ist oder an einem Kohlenstoffatom einfach oder mehrmals mit einem Fluoratom oder mit einem ($C_1$-$C_3$)-Alkyl substituiert ist;
- $R_1$ für Folgendes steht:

. eine -$NR_8R_9$-Gruppe;
. einen Piperidin-3-yl- oder Piperidin-4-ylrest, der unsubstituiert ist oder einfach oder mehrmals mit einem ($C_1$-$C_4$)-Alkyl, einem ($C_3$-$C_5$)-Cycloalkyl substituiert ist, wobei die Kohlenstoffatome auch mit einem oder mehreren Fluoratomen substituiert sein können;

- $R_2$ für ein Wasserstoffatom, ein Halogenatom, eine Alk-Gruppe, eine OAlk-Gruppe steht;
- $R_3$ für ein Wasserstoffatom, ein Halogenatom, eine Alk-Gruppe, eine OAlk-Gruppe steht;
- $R_4$ für ein Wasserstoffatom, ein Halogenatom, eine Alk-Gruppe, ein Hydroxyl, eine OAlk-Gruppe steht;
- $R_5$ für ein Wasserstoffatom, ein Halogenatom, eine Alk-Gruppe, ein Hydroxyl, eine OAlk-Gruppe steht;
- $R_6$ für ein Wasserstoffatom, ein Halogenatom, eine Alk-Gruppe, ein Hydroxyl, eine OAlk-Gruppe steht;
- $R_7$ für ein Wasserstoffatom, ein Halogenatom, eine Alk-Gruppe, ein Hydroxyl, eine OAlk-Gruppe steht;
- $R_8$ und $R_9$ jeweils unabhängig voneinander für ein Wasserstoffatom oder ein ($C_1$-$C_4$)-Alkyl stehen;
- oder $R_8$ und $R_9$ gemeinsam mit dem Stickstoffatom, an welches sie gebunden sind, einen heterozyklischen Rest bilden, der aus den folgenden ausgewählt ist: Azetidin-1-yl, Pyrrolidin-1-yl, Piperidin-1-yl, Morpholin-4-yl, Thiomorpholin-4-yl, Piperazin-1-yl oder Perhydroazepin-1-yl, wobei der heterozyklische Rest unsubstituiert ist oder aber einfach oder mehrmals mit einem Amino, einem Hydroxyl, einem ($C_1$-$C_4$)-Alkyl, einem ($C_3$-$C_5$)-Cycloalkyl, einem ($C_1$-$C_4$)-Alkoxy substituiert ist, wobei die Kohlenstoffatome auch mit einem oder mehreren Fluoratomen substituiert sein können;
- Alk für ein ($C_1$-$C_4$)-Alkyl steht, das unsubstituiert ist oder aber einfach oder mehrmals mit einem Fluoratom substituiert ist.

in Form einer Base oder eines Säureadditionssalzes.

**2.** Verbindungen der Formel (I) nach Anspruch 1, wobei:

- X für einen zweiwertigen ($C_3$-$C_4$)-Alkylenrest steht, der unsubstituiert ist oder an einem Kohlenstoffatom einfach oder mehrmals mit einem Fluoratom oder mit einem ($C_1$-$C_3$)-Alkyl substituiert ist;
- $R_1$ für Folgendes steht:

. eine -$NR_8R_9$-Gruppe;
. einen Piperidin-4-ylrest;

- $R_2$ für eine Alk-Gruppe, eine OAlk-Gruppe steht;
- $R_3$ für ein Wasserstoffatom steht;
- $R_4$ für ein Halogenatom steht;
- $R_5$ für ein Wasserstoffatom steht;
- $R_6$ für ein Halogenatom, eine OAlk-Gruppe steht;
- $R_7$ für ein Wasserstoffatom, eine Alk-Gruppe, eine OAlk-Gruppe steht;

- $R_8$ und $R_9$ gemeinsam mit dem Stickstoffatom, an welches sie gebunden sind, einen heterozyklischen Rest bilden, der aus den folgenden ausgewählt ist: Piperidin-1-yl, Piperazin-1-yl, wobei der heterozyklische Rest unsubstituiert ist oder aber einfach oder zweifach mit einem Fluoratom, einem Amino, einem Hydroxyl, einem Methyl substituiert ist;
- Alk für ein ($C_1$-$C_4$)-Alkyl steht, das unsubstituiert ist oder aber einfach oder mehrmals mit einem Fluoratom substituiert ist;

in Form einer Base oder eines Säureadditionssalzes.

**3.** Verbindungen der Formel (I) nach Anspruch 1, wobei:

- X für einen zweiwertigen Trimethylen- oder Tetramethylenrest steht;
- $R_1$ für Folgendes steht:

  . ein Piperidin-1-yl, ein Piperazin-1-yl, ein 4-Methylpiperazin-1-yl;
  . ein Piperidin-4-yl;

- $R_2$ für ein Methyl, ein Methoxy steht;
- $R_3$ für ein Wasserstoffatom steht;
- $R_4$ für ein Fluoratom steht;
- $R_5$ für ein Wasserstoffatom steht;
- $R_6$ für ein Chloratom, ein Methoxy, ein Isopropoxy, ein Difluormethoxy steht;
- $R_7$ für ein Wasserstoffatom, ein Methyl, ein Methoxy steht;

in Form einer Base oder eines Säureadditionssalzes.

**4.** Verbindung nach Anspruch 1 der Formel (I), ausgewählt aus:

- 3-(2-Fluorphenyl)-1-[(4-isopropoxyphenyl)-sulfonyl]-5-methoxy-3-(3-piperazin-1-ylpropyl)-1,3-dihydro-2*H*-pyrrolo[3,2-*b*]pyridin-2-on;
- 3-(2-Fluorphenyl)-1-[(4-isopropoxyphenyl)-sulfonyl]-5-methoxy-3-(3-piperazin-1-ylpropyl)-1,3-dihydro-2*H*-pyrrolo[3,2-b]pyridin-2-on;
- 3-(2-Fluorphenyl)-5-methoxy-1-[(3-methoxyphenyl)sulfonyl]-3-(3-piperazin-1-ylpropyl)-1,3-dihydro-2*H*-pyrrolo[3,2-*b*]pyridin-2-on;
- 1-[(3-Chlorphenyl)sulfonyl]-3-(2-fluorphenyl)-5-methoxy-3-(3-piperazin-1-ylpropyl)-1,3-dihydro-2*H*-pyrrolo [3,2-*b*]pyridin-2-on;
-3-(2-Fluorphenyl)-1-[(4-isopropoxyphenyl)-sulfonyl]-5-methoxy-3-(3-piperidin-1-ylpropyl)-1,3-dihydro-2*H*-pyrrolo[3,2-*b*]pyridin-2-on;
-3-(2-Fluorphenyl)-1-[(4-isopropoxyphenyl)-sulfonyl]-5-methoxy-3-(3-piperidin-1-ylpropyl)-1,3-dihydro-2*H*-pyrrolo[3,2-b]pyridin-2-on;
- 3-(2-Fluorphenyl)-5-methoxy-1-[(3-methoxyphenyl)sulfonyl]-3-(3-piperidin-1-ylpropyl)-1,3-dihydro-2*H*-pyrrolo[3,2-b]pyridin-2-on, Isomer;
- 1-{[4-(Difluormethoxy)phenyl]sulfonyl}-3-(2-fluorphenyl)-5-methoxy-3-(3-piperidin-1-ylpropyl)-1,3-dihydro-2*H*-pyrrolo[3,2-*b*]pyridin-2-on;
- 1-[(3-Chlorphenyl)sulfonyl]-3-(2-fluorphenyl)-5-methoxy-3-(3-piperidin-1-ylpropyl)-1,3-dihydro-2*H*-pyrrolo [3,2-*b*]pyridin-2-on;
- 3-(2-Fluorphenyl)-1-[(4-isopropoxyphenyl)-sulfonyl]-5-methoxy-3-(4-piperazin-1-ylbutyl)-1,3-dihydro-2*H*-pyrrolo[3,2-*b*]pyridin-2-on;
- 3-(2-Fluorphenyl)-1-[(4-isopropoxyphenyl)-sulfonyl]-5-methoxy-3-[3-(4-methylpiperazin-1-yl)propyl]-1,3-dihydro-2*H*-pyrrolo[3,2-*b*]pyridin-2-on;
- 3-(2-Fluorphenyl)-1-[(4-isopropoxyphenyl)-sulfonyl]-5-methoxy-3-[4-(4-methylpiperazin-1-yl)butyl]-1,3-dihydro-2*H*-pyrrolo[3,2-*b*]pyridin-2-on;
- 3-(2-Fluorphenyl)-1-[(4-isopropoxyphenyl)-sulfonyl]-5-methoxy-3-(3-piperidin-4-yl-propyl)-1,3-dihydro-2*H*-pyrrolo[3,2-*b*]pyridin-2-on;
- 3-(2-Fluorphenyl)-1-[(4-isopropoxyphenyl)-sulfonyl]-5-methoxy-3-(4-piperazin-1-ylbutyl)-1,3-dihydro-2*H*-pyrrolo[3,2-*b*]pyridin-2-on;
- 3-(2-Fluorphenyl)-1-[(4-isopropoxyphenyl)-sulfonyl]-5-methyl-3-(3-piperazin-1-yl-propyl)-1,3-dihydro-2*H*-pyrrolo[3,2-b]pyridin-2-on;
- 3-(2-Fluorphenyl)-5-methoxy-1-[(4-methoxy-3-methylphenyl)sulfonyl]-3-(4-piperazin-1-ylbutyl)-1,3-dihydro-2*H*-pyrrolo[3,2-*b*]pyridin-2-on;

- 1-[(2,4-Dimethoxyphenyl)sulfonyl]-3-(2-fluorphenyl)-5-methyl-3-(3-piperazin-1-ylpropyl)-1,3-dihydro-2*H*-pyrrolo[3,2-*b*]pyridin-2-on;
- 3-(2-Fluorphenyl)-5-methoxy-1-[(4-methoxy-3-methylphenyl)sulfonyl]-3-(3-piperazin-1-ylpropyl)-1,3-dihydro-2*H*-pyrrolo[3,2-*b*]pyridin-2-on;
- 1-[(2,4-Dimethoxyphenyl)sulfonyl]-3-(2-fluorphenyl)-5-methoxy-3-(3-piperazin-1-ylpropyl)-1,3-dihydro-2*H*-pyrrolo[3,2-*b*]pyridin-2-on;
- 3-(2-Fluorphenyl)-1-[(4-methoxy-3-methylphenyl)sulfonyl]-5-methyl-3-(3-piperazin-1-ylpropyl)-1,3-dihydro-2*H*-pyrrolo[3,2-*b*]pyridin-2-on;
- 1-[(2,4-Dimethoxyphenyl)sulfonyl]-3-(2-fluorphenyl)-5-methoxy-3-(4-piperazin-1-ylbutyl)-1,3-dihydro-2*H*-pyrrolo[3,2-*b*]pyridin-2-on;
- 3-(2-Fluorphenyl)-1-[(4-isopropoxyphenyl)-sulfonyl]-5-methyl-3-[3-(4-methylpiperazin-1-ylpropyl]-1,3-dihydro-2*H*-pyrrolo[3,2-*b*]pyridin-2-on;
- 3-(2-Fluorphenyl)-5-methoxy-1-[(4-methoxy-3-methylphenyl)sulfonyl]-3-[4-(4-methylpiperazin-1-yl)butyl)-1,3-dihydro-2*H*-pyrrolo[3,2-*b*]pyridin-2-on;

in Form einer Base oder eines Säureadditionssalzes; in Form reiner Enantiomere oder in Racematmischung.

**5.** Verfahren zur Herstellung von Verbindungen der Formel (I) nach einem beliebigen der Ansprüche 1 bis 4, **dadurch gekennzeichnet, dass**:

eine Verbindung der Formel:

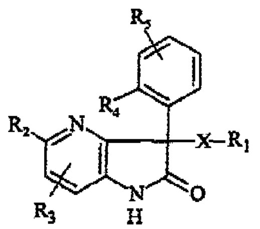 (II)

wobei X, $R_1$, $R_2$, $R_3$, $R_4$ und $R_5$ den Begriffsbestimmungen für eine Verbindung der Formel (I) entsprechen, in Gegenwart einer Base mit einem Sulfonylhalogenid der Formel:

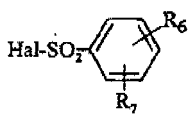 (III)

wobei $R_6$ und $R_7$ den Begriffsbestimmungen für eine Verbindung der Formel (I) entsprechen und Hal für ein Halogenatom steht, umgesetzt wird.

**6.** Arzneimittel, **dadurch gekennzeichnet, dass** es eine Verbindung der Formel (I) nach einem beliebigen der Ansprüche 1 bis 4 oder ein Additionssalz dieser Verbindung mit einer pharmazeutisch unbedenklichen Säure umfasst.

**7.** Pharmazeutische Zusammensetzung, **dadurch gekennzeichnet, dass** sie eine Verbindung der Formel (I) nach einem beliebigen der Ansprüche 1 bis 4 oder ein pharmazeutisch unbedenkliches Salz sowie mindestens einen pharmazeutisch unbedenklichen Trägerstoff umfasst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9315051 A
- EP 636608 A
- EP 636609 A
- WO 9715556 A
- WO 9825901 A
- WO 0155130 A
- WO 0155134 A
- WO 0164668 A
- WO 0198295 A
- WO 03008407 A
- WO 06080574 A
- WO 08025735 A
- WO 9518105 A
- WO 95018105 A
- EP 0469984 B


### Littérature non-brevet citée dans la description


- Vasopressin and oxytocin receptors : an overview. **S. JARD et al.** Progress in Endocrinology. Experta Medica, 1988, 1183-1188
- *J. Lab. Clin. Med.,* 1989, vol. 114 (6), 617-632
- *Pharmacol. Rev.,* 1991, vol. 43 (1), 73-108
- **G. GUILLON et al.** *Endocrinology,* 1995, vol. 136 (3), 1285-1295
- **G. MAZZOCCHI et al.** *Peptides,* 1997, vol. 18 (2), 191-195
- **E. GRAZZINI et al.** *J. Clin. Endocrinol. Metab.,* 1999, vol. 84 (6), 2195-2203
- **P.J. WOLL et al.** *Biochem. Biophys. Res. Commun.,* 1989, vol. 164 (1), 66-73
- **S. Jard et al.** *Mol. Pharmacol.,* 1986, vol. 30, 171-177
- **Y. Arsenijevic et al.** *J. Endocrinol.,* 1994, vol. 141, 383-391
- **J. Schwartz et al.** *Endocrinology,* 1991, vol. 129 (2), 1107-1109
- **Y. de Keyser et al.** *FEBS Letters,* 1994, vol. 356, 215-220
- **G. E. Gdjjes et al.** *Nature,* 1982, vol. 299, 355
- *Neuroendocrinology,* 1994, vol. 60, 503-508
- **Y. de Keyser.** *FEBS Letters,* 1994, vol. 356, 215-220
- **T. Sugimoto et al.** *J. Biol. Chem.,* 1994, vol. 269 (43), 27088-27092
- **M. Saito et al.** *Biochem. Biophys. Res. Commun.,* 1995, vol. 212 (3), 751-757
- **S. J. Lolait et al.** *Neurobiology,* 1996, vol. 92, 6783-6787
- **M. A. Ventura et al.** *Journal of Molecular Endocrinology,* 1999, vol. 22, 251-260
- **B. Lee et al.** Endocrinol. Metab. *Am. J. Physiol. 269,* 1995, vol. 32
- **E. Grazzini et al.** *Endocrinology,* 1996, vol. 137 (a), 3906-3914
- **J. Bertherat et al.** *Eur. J. Endocrinol.,* 1996, vol. 135, 173
- **G. A. Wuinert et al.** *Lancet,* 1990, vol. 335, 991-994
- **G. Dickstein et al.** *J. Clin. Endocrinol. Metab.,* 1996, vol. 81 (8), 2934-2941
- Molecular cloning and functional expression of V1b receptor gene. **T. Sugimoto et al.** Neurohypophysis : Recent Progress of Vasopressin and Oxytocin Research. Elsevier Science, 1995, 409413
- **Green et al.** Protective Group in Organic Synthesis. John Wiley & Sons, Inc, 2007
- **M. B. Smith ; J. March.** Advanced Organic Chemistry. Wiley Interscience, 2007, 496-501
- **R J. Cremlyn.** Chlorosulfonic Acid. The Royal Society of Chemistry, 2002
- **M. Thibonnier et al.** *J. Biol. Chem.,* 1994, vol. 269, 3304-3310
- **Y. de Keyser et al.** *Febs Letters,* 1994, vol. 356, 215-220
- **M. Birnbaumer et al.** *Nature (Lond.),* 1992, vol. 357, 333-335
- **J. Elands et al.** *Eur. J. Pharmacol.,* 1987, vol. 147, 197-207
- **Sullivan et al.** *Methods Mol. Biol.,* 1999, vol. 114, 125-133
- *J. Clin. Invest.,* 1993, vol. 92, 224-231